# EUROPÄISCHE PATENTSCHRIFT

(11) **EP 2 606 042 B1**
(45) Veröffentlichungstag und Bekanntmachung des Hinweises auf die Patenterteilung: **03.09.2014**
(21) Anmeldenummer: 11748634.0
(22) Anmeldetag: 19.08.2011
(51) Int. Cl.: C07D 401/12, C07D 401/14, C07D 403/12, A61K 31/455, A61K 31/506, A61K 31/501, A61P 29/00, A61P 11/00, A61P 19/02, A61P 17/00

(54) **DISUBSTITUIERTE TETRAHYDOFURANYL-VERBINDUNGEN ALS ANTAGONISTEN DES BRADYKININ-B1-REZEPTORS**
DISUBSTITUTED TETRAHYDROFURANYL COMPOUNDS AS BRADYKININ B1 RECEPTOR ANTAGONISTS
COMPOSÉS DE TÉTRAHYDROFURANYL DISUBSTITUÉS EN TANT QU'ANTAGONISTES DU RÉCEPTEUR B1 DE LA BRADYKININE

(30) Priorität: 20.08.2010 EP 10173489
(43) Veröffentlichungstag der Anmeldung: 26.06.2013
(73) Patentinhaber: Boehringer Ingelheim International GmbH, 55216 Ingelheim am Rhein (DE)
(72) Erfinder: HAUEL, Norbert, 55216 Ingelheim Am Rhein (DE); CECI, Angelo, 55216 Ingelheim Am Rhein (DE); DOODS, Henri, 55216 Ingelheim Am Rhein (DE); JUNG, Birgit, 55216 Ingelheim Am Rhein (DE); KUELZER, Raimund, 55216 Ingelheim Am Rhein (DE)
(74) Vertreter: Simon, Elke Anna Maria
(86) Internationale Anmeldenummer: PCT/EP2011/064260
(87) Internationale Veröffentlichungsnummer: WO 2012/022795

(56) Entgegenhaltungen:
- WO-A1-2005/016886
- WO-A1-2009/027450
- WO-A1-2010/057899

## Beschreibung

Gegenstand der vorliegenden Erfindung sind disubstituierte Tetrahydrofuranyl-Verbindungen der allgemeinen Formel I

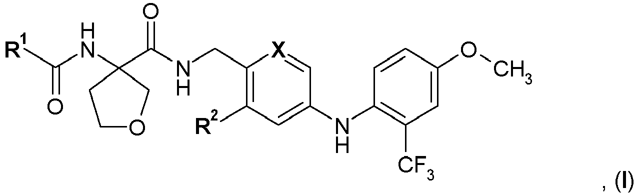

in der die Variablen **R¹**, **R²** und **X** wie nachstehend beschrieben definiert sind, deren Enantiomere, deren Diastereomere, deren Gemische und deren Salze, insbesondere deren physiologisch verträgliche Salze mit organischen oder anorganischen Säuren oder Basen, welche wertvolle Eigenschaften aufweisen, Verfahren zu deren Herstellung, die die pharmakologisch wirksamen Verbindungen enthaltenden Arzneimittel sowie deren Herstellung und deren Verwendung.

### HINTERGRUND DER ERFINDUNG

### TECHNISCHES GEBIET

Die vorliegende Erfindung betrifft disubstituierte Tetrahydrofuranyl-Verbindungen und deren Verwendung als B1-Rezeptor-Antagonisten, pharmazeutische Zusammensetzungen enthaltend diese Verbindungen sowie Methoden zur Verwendung derselben zur Vorbeugung oder Behandlung von akuten Schmerzen, Eingeweideschmerzen, neuropathischen Schmerzen, entzündlichen und Schmerzrezeptor-vermittelten Schmerzen, Tumorschmerzen sowie Kopfschmerzen.

### STAND DER TECHNIK

In den Patentanmeldungen WO 2009/027450, WO 2005/016886 und WO 2010/057899 werden bereits Verbindungen mit B1-antagonistischer Wirkung beschrieben.

Eine Aufgabe der vorliegenden Erfindung bestand darin, neue Verbindungen zur Verfügung zu stellen, die sich insbesondere als pharmakologische Wirkstoffe in Arzneimitteln eignen, die zur Behandlung von Krankheiten verwendet werden können, welche zumindest teilweise durch den B1-Rezeptor vermittelt werden.

### DETAILLIERTE BESCHREIBUNG DER ERFINDUNG

In der obigen allgemeinen Formel I bedeuten in einer Ausführungsform 1
- **R¹**: eine Gruppe ausgewählt aus 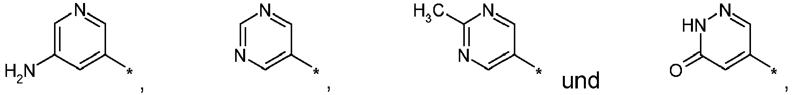
- **R²**: H, Cl oder F und
- **X**: CH oder N,
deren Enantiomere, deren Diastereomere, deren Gemische und deren Salze, insbesondere deren physiologisch verträgliche Salze mit organischen oder anorganischen Säuren oder Basen.

Eine Ausführungsform **2** der vorliegenden Erfindung besteht in den Verbindungen der allgemeinen Formel **Ia** 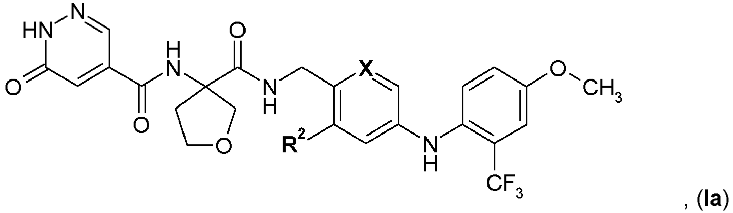 in der
- **R²**: H, Cl oder F und
- **X**: CH oder N bedeuten,
deren Enantiomere, deren Diastereomere, deren Gemische und deren Salze, insbesondere deren physiologisch verträgliche Salze mit organischen oder anorganischen Säuren oder Basen.

Eine Ausführungsform **3** der vorliegenden Erfindung besteht in den Verbindungen der allgemeinen Formel **Ib** 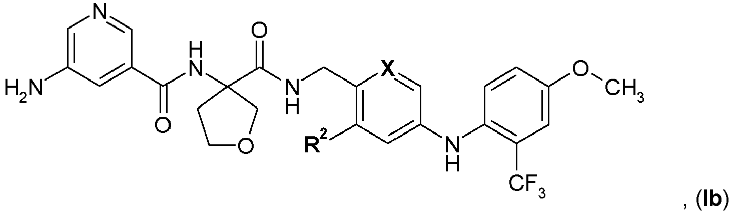 in der
- **R²**: H, Cl oder F und
- **X**: CH oder N bedeuten,
deren Enantiomere, deren Diastereomere, deren Gemische und deren Salze, insbesondere deren physiologisch verträgliche Salze mit organischen oder anorganischen Säuren oder Basen.

Eine Ausführungsform **4** der vorliegenden Erfindung besteht in den Verbindungen der allgemeinen Formel **Ic** 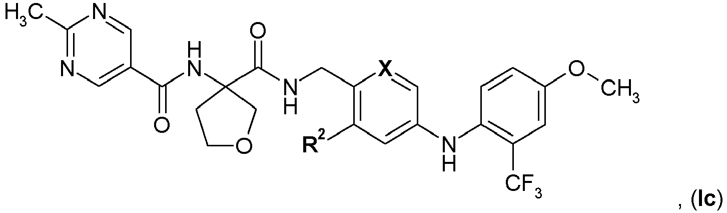 in der
- **R²**: H, Cl oder F und
- **X**: CH oder N bedeuten,
deren Enantiomere, deren Diastereomere, deren Gemische und deren Salze, insbesondere deren physiologisch verträgliche Salze mit organischen oder anorganischen Säuren oder Basen.

Eine Ausführungsform **5** der vorliegenden Erfindung besteht in den Verbindungen der allgemeinen Formel **Id** 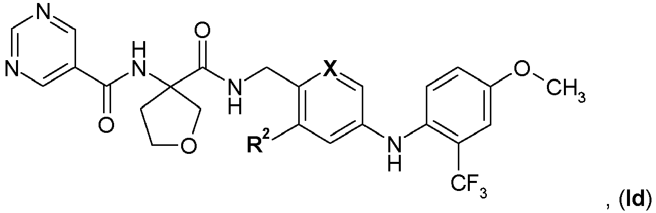 in der
- **R²**: H, Cl oder F und
- **X**: CH oder N bedeuten,
deren Enantiomere, deren Diastereomere, deren Gemische und deren Salze, insbesondere deren physiologisch verträgliche Salze mit organischen oder anorganischen Säuren oder Basen.

Als ganz besonders bevorzugte Verbindungen der obigen allgemeinen Formel **I** seien beispielsweise folgende genannt:

| **Nr.** | **Struktur** |
|---|---|
| (1) | 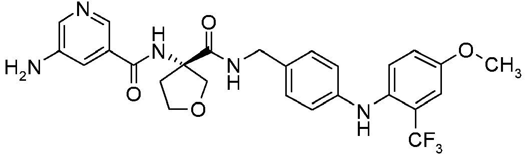 |
| (2) | 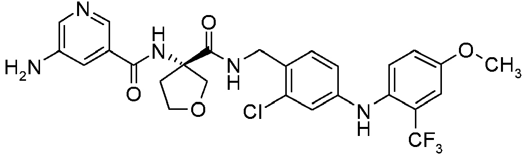 |
| (3) | 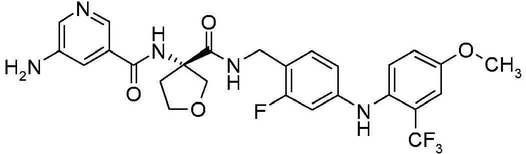 |
| (4) | 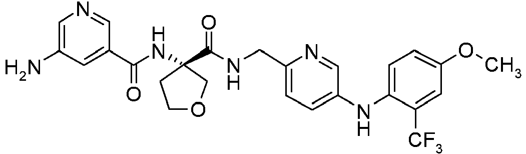 |
| (5) | 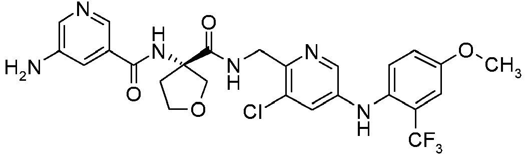 |
| (6) | 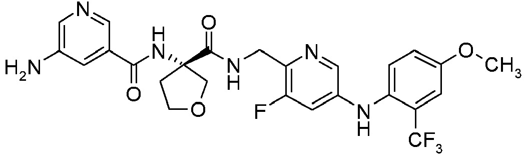 |
| (7) | 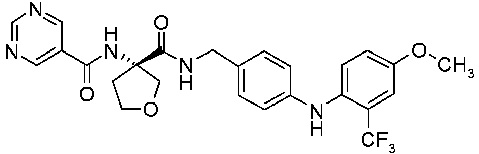 |
| (8) | 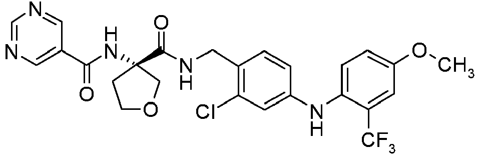 |
| (9) | 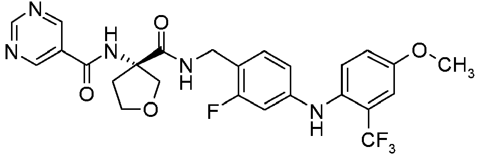 |
| (10) | 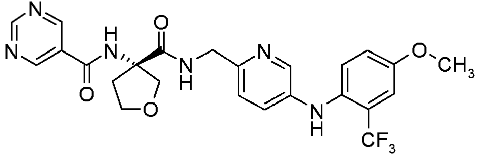 |
| (11) | 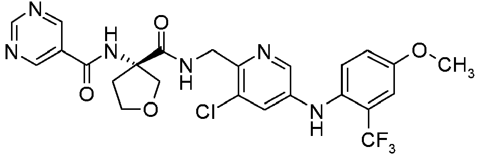 |
| (12) | 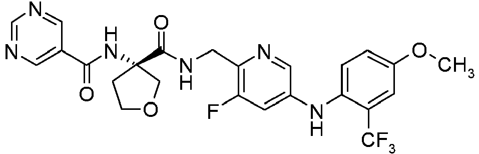 |
| (13) | 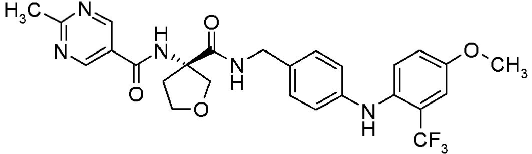 |
| (14) | 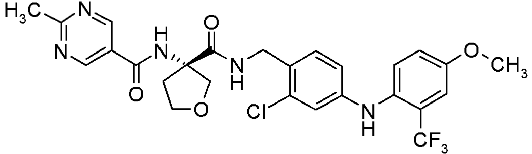 |
| (15) | 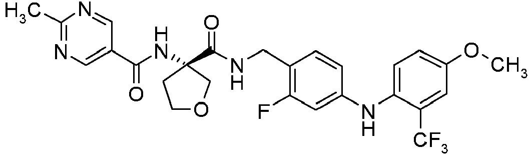 |
| (16) | 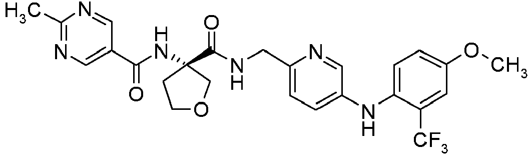 |
| (17) | 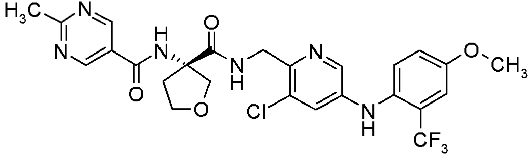 |
| (18) | 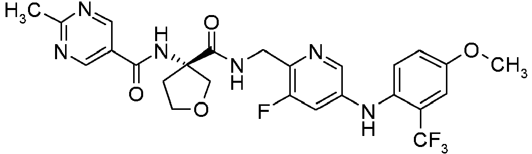 |
| (19) | 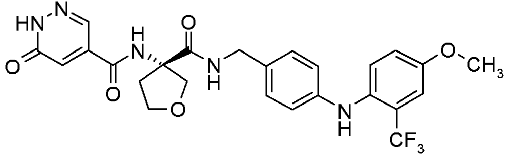 |
| (20) | 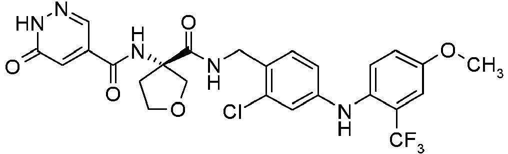 |
| (21) | 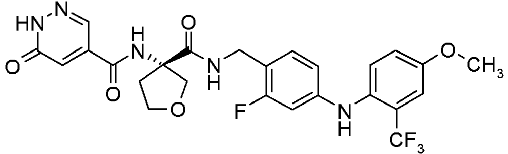 |
| (22) | 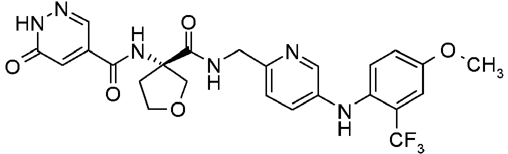 |
| (23) | 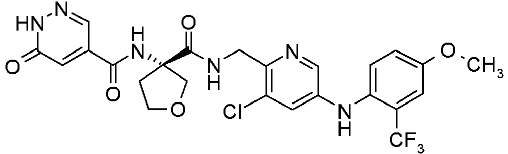 |
| (24) | 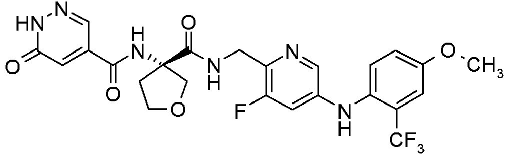 |

deren Enantiomere, deren Diastereomere, deren Gemische und deren Salze, insbesondere deren physiologisch verträgliche Salze mit organischen oder anorganischen Säuren oder Basen.

### VERWENDETE BEGRIFFE UND DEFINITIONEN

Vom Gegenstand dieser Erfindung umfasst sind die erfindungsgemäßen Verbindungen der eingangs erwähnten allgemeinen Formel **I**, einschließlich deren Salze, in denen ein oder mehrere Wasserstoffatome, beispielsweise ein, zwei, drei, vier oder fünf Wasserstoffatome, durch Deuterium ausgetauscht sind.

Soweit nicht anders angegeben, umfasst eine in der Beschreibung oder in einem Anspruch angegebene chemische Formel oder ein Name sowohl alle strukturell möglichen und thermodynamisch stabilen Tautomere als auch alle Stereoisomere, optischen Isomere, geometrische Isomere (z.B. Enantiomere, Diastereomere, E/Z-Isomere etc.), Racemate sowie Mischungen unterschiedlicher Anteile der einzelnen Enantiomere, Mischungen von Diastereomeren oder Mischungen jeder voranstehend erwähnten Form, bei der Isomere und Enantiomere existieren.

Ebenfalls vom Gegenstand der Erfindung umfasst sind Solvate der Verbindungen der allgemeinen Formel **I**, beispielsweise deren Hydrate.

Weiterhin vom Gegenstand der Erfindung umfasst sind die Salze der jeweils genannten Verbindungen, einschließlich der physiologisch verträglichen Salze, sowie deren Solvate, wie beispielsweise Hydrate.

Verbindungen der allgemeinen Formel **I** können, sofern sie geeignete basische Funktionen enthalten, beispielsweise Aminogruppen, insbesondere für pharmazeutische Anwendungen in ihre physiologisch verträglichen Salze mit anorganischen oder organischen Säuren übergeführt werden.

Unter dem Begriff "physiologisch verträgliches Salz" versteht man im Sinne der vorliegenden Erfindung vorzugsweise Salze der erfindungsgemäßen Verbindungen, die physiologisch verträglich sind, d.h. insbesondere zur Anwendung am Menschen und/oder Säugetier geeignet sind.

Der Begriff "physiologisch verträglich" wird im Sinner der vorliegenden Erfindung als Hinweis auf diejenigen Verbindungen, Inhaltsstoffe, Zusammensetzungen und/oder Darreichungsformen verwendet, welche im Rahmen einer vernünftigen medizinischen Beurteilung zur Verwendung im Kontakt mit menschlichem oder tierischem Gewebe geeignet sind, ohne übermäßige Toxizität, Irritationen, allergische Reaktionen oder andere Probleme oder Komplikationen, und die einem angemessenen Nutzen-Risiko-Verhältnis entsprechen.

Als anorganische Säuren kommen hierfür beispielsweise Bromwasserstoffsäure, Phosphorsäure, Salpetersäure, Salzsäure oder Schwefelsäure in Frage, als organische Säuren kommen beispielsweise Ameisensäure, Äpfelsäure, Ascorbinsäure, Benzoesäure, Bernsteinsäure, Essigsäure, Ethylendiamintetraessigsäure, Fumarsäure, Glutaminsäure, Hexan-1-sulfonsäure, Kohlensäure, Maleinsäure, Mandelsäure, Milchsäure, Monomethylsebacinsäure, Nicotinsäure, Oxalsäure, 5-Oxoprolin, Saccharinsäure, Sulfonsäuren, wie beispielsweise Methansulfonsäure, Camphersulfonsäure, Ethansulfonsäure, Ethan-1,2-disulfonsäure, Benzolsulfonsäure oder *p*-Toluolsulfonsäure, Weinsäure oder Zitronensäure in Betracht (siehe "Pharmaceutical salts", Birge, S.M. et al., J. Pharm. Sci., (1977), 66, 1-19).

Weiterhin lassen sich die Verbindungen der allgemeinen Formel **I**, sofern sie geeignete Carbonsäurefunktionen enthalten, insbesondere für pharmazeutische Anwendungen in ihre physiologisch verträglichen Salze mit anorganischen oder organischen Basen überführen. Als anorganische Basen kommen hierfür beispielsweise Alkali- oder Erdalkalimetallhydroxide, beispielsweise Natriumhydroxid oder Kaliumhydroxid, oder Carbonate, Ammoniak, Zink- oder Ammoniumhydroxide in Frage; als organische Amine kommen beispielsweise Diethylamin, Triethylamin, Ethanolamin, Diethanolamin, Triethanolamin, Cyclohexylamin oder Dicyclohexylamin in Betracht.

Die physiologisch verträglichen Salze gemäß der vorliegenden Erfindung können ausgehend von den erfindungsgemäßen Verbindungen, welche eine geeignete basische oder saure Einheit enthalten, über herkömmliche chemische Methoden synthetisiert werden. Generell können solche Salze durch Reaktion der freien Säure- oder Basegruppe mit einer notwendigen Menge an Base oder Säure in Wasser oder einem organischen Lösungsmittel, wie beispielsweise Diethylether, Ethylacetat, Ethanol, Isopropanol, Acetonitril oder einer Mischung dieser Lösungsmittel, hergestellt werden.

Die erfindungsgemäßen Verbindungen können als Racemate vorliegen, sofern sie nur ein Chiralitätselement besitzen, sie können aber auch als reine Enantiomere, d.h. in (R)- oder (S)-Form gewonnen werden.

Die Anmeldung umfasst jedoch auch die einzelnen diastereomeren Antipodenpaare oder deren Gemische, die dann vorliegen, wenn mehr als ein Chiralitätselement in den Verbindungen der allgemeinen Formel **I** vorhanden ist, sowie die einzelnen optisch aktiven Enantiomeren, aus denen sich die erwähnten Racemate zusammensetzen.

Verbindungen mit einer Kohlenstoff-Doppelbindung können sowohl in der E- als auch Z-Form vorliegen.

Falls eine Verbindung in verschiedenen tautomeren Formen vorliegen kann, so ist die dargestellte Verbindung nicht auf eine tautomere Form beschränkt, sondern umfasst alle tautomeren Formen. Dies gilt insbesondere auch bei stickstoffhaltigen Heteroarylen:

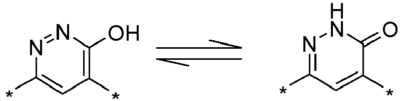

### HERSTELLVERFAHREN

Erfindungsgemäß erhält man die Verbindungen der allgemeinen Formel **I** nach an sich bekannten Verfahren, beispielsweise nach folgenden Verfahren:

### (A) Amid-Kupplung:

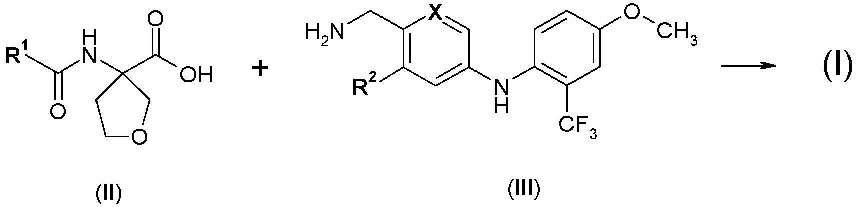

Die dargestellte Verknüpfung von Carbonsäuren der allgemeinen Formel **II**, in der **R¹** wie vorstehend erwähnt definiert ist, mit Aminen der allgemeinen Formel **III**, in der **R²** und **X** wie vorstehend erwähnt definiert sind, unter Bildung von Carbonsäureamiden der allgemeinen Formel **I**, in der **R¹**, **R²** und **X** wie vorstehend erwähnt definiert sind, kann mit herkömmlichen Methoden zur Amidbildung durchgeführt werden.

Die Kupplung wird bevorzugt unter Verwendung von aus der Peptidchemie bekannten Verfahren (siehe z. B. Houben-Weyl, Methoden der Organischen Chemie, Bd. 15/2) durchgeführt, wobei zum Beispiel Carbodiimide, wie z.B. Dicyclohexylcarbodiimid (DCC), Diisopropylcarbodiimid (DIC) oder Ethyl-(3-dimethylamino-propyl)-carbodiimid, *O*-(1H-Benzotriazol-1-yl)-*N*,*N*-*N'*,*N'*-tetramethyluronium-hexafluorphosphat (HBTU) oder -tetrafluorborat (TBTU) oder 1*H*-Benzotriazol-1-yl-oxy-tris-(dimethylamino)-phosphonium-hexafluorphosphat (BOP) eingesetzt werden. Durch Zugabe von 1-Hydroxybenzotriazol (HOBt) oder von 3-Hydroxy-4-oxo-3,4-di-hydro-1,2,3-benzotriazin (HOObt) kann die Reaktionsgeschwindigkeit gesteigert werden. Die Kupplungen werden normalerweise mit äquimolaren Anteilen der Kupplungskomponenten sowie des Kupplungsreagenz in Lösungsmitteln wie Dichlormethan, Tetrahydrofuran (THF), Acetonitril, Dimethylformamid (DMF), Dimethylacetamid (DMA), *N*-Methylpyrrolidon (NMP) oder Gemischen aus diesen durchgeführt. Sofern erforderlich wird zusätzlich eine Hilfsbase wie beispielsweise Diisopropylethylamin (DIPEA, Hünig-Base) eingesetzt.

Weiter ist es möglich, die Carbonsäuren der allgemeinen Formel **II**, in der **R¹** wie vorstehend erwähnt definiert ist, in die entsprechenden Carbonsäurechloride zu überführen und diese anschließend mit Aminen der allgemeinen Formel **III**, in der **R²** und **X** wie vorstehend erwähnt definiert sind, umzusetzen. Die Synthese von Carbonsäurechloriden erfolgt nach literaturbekannten Verfahren (siehe z. B. Houben-Weyl, Methoden der Organischen Chemie, Bd. E5/1).

### (B) Reduktion der Nitrilgruppe:

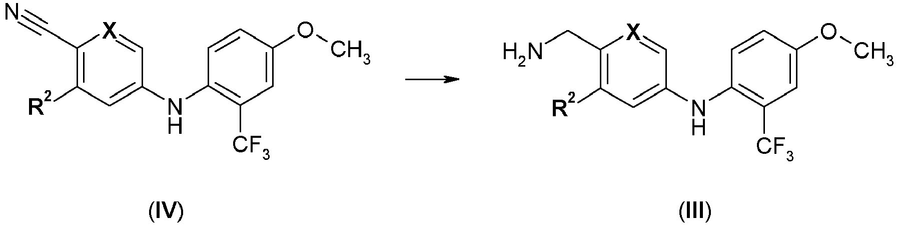

Die Reduktion eines Nitrils der allgemeinen Formel **IV**, in der **R²** und **X** wie vorstehend erwähnt definiert sind, zu einem Amin der allgemeinen Formel **III**, in der **R²** und **X** wie vorstehend erwähnt definiert sind, kann unter Standard-Bedingungen der katalytischen Hydrogenolyse mit einem Katalysator, wie beispielsweise Raney-Nickel, in einem Lösungsmittel, wie beispielsweise ammoniakalisches Methanol oder Ethanol, oder mit einem Reduktionsmittel, wie beispielsweise Lithiumaluminiumhydrid oder Natriumborhydrid, in einem Lösungsmittel, wie beispielsweise Tetrahydrofuran, gegebenenfalls in Gegenwart einer Lewissäure wie Aluminiumchlorid, durchgeführt werden.

### (C) nucleophile aromatische Substitution oder Übergangsmetall-katalysierte Kupplung:

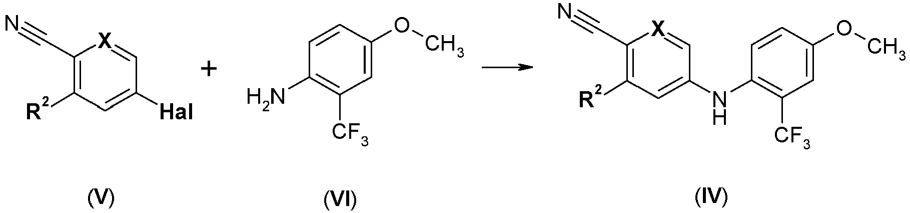

Die Reaktion des Anilins **VI** mit einem Nitril der allgemeinen Formel **V**, in der **R²** und **X** wie vorstehend erwähnt definiert sind und **Hal** ein Fluor-, Chlor- oder Bromatom bedeutet, erfolgt nach bekannten Verfahren, beispielsweise ohne Lösungsmittel oder in einem Lösungsmittel wie Tetrahydrofuran, Dimethylformamid oder Dimethylsulfoxid und zweckmäßigerweise in Gegenwart einer Base, wie beispielsweise Triethylamin, Natronlauge oder Kaliumcarbonat, bei einer Temperatur von 20°C bis 160°C.

Eine alternative Methode zur Darstellung von Verbindungen der allgemeinen Formel **IV** stellt die Palladium katalysierte Reaktion eines Nitrils der allgemeinen Formel V, in dem **Hal** Chlor, Brom oder Jod bedeutet, mit dem Anilin VI dar. Reaktionsbedingungen für diese auch als Buchwald-Hartwig Reaktion bekannten Umsetzung sind aus der Literatur bekannt.

### Methodenbeschreibung zur cynoBK1-Rezeptorbindung

CHO -Zellen, die den Cynomolgus B1-Rezeptor exprimieren, werden in "HAM'S F-12 Medium" kultiviert. Von konfluenten Kulturen wird das Medium entfernt, die Zellen werden mit PBS-Puffer gewaschen, abgeschabt oder mit Versene abgelöst und durch Zentrifugieren isoliert. Anschließend werden die Zellen in Suspension homogenisiert, das Homogenat zentrifugiert und resuspendiert. Nach Bestimmung des Proteingehalts werden 200 µl des Homogenats (50 bis 250 µg Protein/Assay) für 60-180 Minuten bei Raumtemperatur mit 0.5 bis 5.0 nM Kallidin (DesArg10,Leu9), [3,4-Prolyl-3,43H(N)] und ansteigenden Konzentrationen der Testsubstanz in einem Gesamtvolumen von 250 µl inkubiert. Die Inkubation wird durch rasche Filtration durch GF/B-Glasfaserfilter, die mit Polyethylenimin (0.3%) vorbehandelt wurden, beendet. Die an das Protein gebundene Radioaktivität wird mit einem TopCount NXT gemessen. Als nichtspezifische Bindung wird die gebundene Radioaktivität in Gegenwart von 1.0 µM (DesArg10) Kallidin definiert. Die Analyse der Konzentrations-Bindungskurve kann mit Hilfe einer computergestützten nichtlinearen Kurvenanpassung erfolgen, um für die Testsubstanz den entsprechenden Kᵢ-Wert zu ermittelt.

Testergebnisse vom cynoBK1-Rezeptorbindungsassay:

| **Beispiel Nr.** | **Kᵢ [nM]** |
|---|---|
| (1) | 1.0 |
| (3) | 4.7 |
| (4) | 3.6 |
| (6) | 18 |
| (21) | 1.6 |

### INDIKATIONEN

Aufgrund ihrer pharmakologischen Eigenschaften eignen sich die neuen Verbindungen und deren physiologisch verträgliche Salze zur Behandlung von Krankheiten und Krankheitssymptomen, die wenigstens teilweise durch die Stimulierung von Bradykinin-B1-Rezeptoren hervorgerufen werden, oder in denen eine Antagonisierung des Bradykinin-1 Rezeptors eine Symptomverbesserung bewirken kann.

Ein weiterer Gegenstand der vorliegenden Erfindung umfasst die erfindungsgemäßen Verbindungen der eingangs erwähnten allgemeinen Formel **I** zur Verwendung als Arzneimittel.

Aufgrund ihrer pharmakologischen Wirkung eignen sich die Substanzen zur Behandlung von
(a) **akuten Schmerzen**, wie beispielsweise Zahnschmerzen, peri- und postoperativen Schmerzen, traumatischen Schmerzen, Muskelschmerzen, Verbrennungsschmerzen, Schmerzen bei Sonnenbrand, Trigeminusneuralgie, Schmerzen bei Koliken, sowie bei Spasmen des Magen-Darm-Trakts oder des Uterus;
(b) **Eingeweideschmerzen**, wie beispielsweise chronischen Beckenschmerzen, gynäkologischen Schmerzen, Schmerzen vor und während der Menstruation, Schmerzen bei Pankreatitis, bei peptischen Ulzera, bei interstitieller Zystitis, bei Nierenkolik, Cholecystitis, Prostatitis, bei Angina pectoris, Schmerzen bei Kolon irritabile, bei nichtulzeröser Dyspepsie und bei Gastritis, Prostatitis, nicht-kardialen Thoraxschmerzen und Schmerzen bei myokardialer Ischämie und Herzinfarkt;
(c) **neuropathischen Schmerzen**, wie beispielsweise schmerzhaften Polyneuropathien, Schmerzen bei diabetischer Neuropathie, AIDS-assoziierten neuropathischen Schmerzen, bei nicht-Herpes-assoziierter Neuralgie, bei Post-Zoster Neuralgie, bei Nervenverletzungen, bei Schädel-Hirn-Trauma, Schmerzen bei Nervenschädigungen infolge von Toxinen oder Chemotherapie, Phantomschmerzen, Schmerzen bei multipler Sklerose, bei Nervenwurzelabriss und schmerzhaften traumatisch bedingten Einzelnervenschädigungen, sowie zentralem Schmerz wie z.B. nach Schlaganfall, Rückenmarksverletzungen oder Tumoren;
(d) **entzündlichen / Schmerzrezeptor-vermittelten Schmerzen** im Zusammenhang mit Erkrankungen wie beispielsweise Osteoarthritis, rheumatoider Arthritis, rheumatischem Fieber, Tendo-Synovitis, Bursitis, Sehnenentzündungen, Gicht und Gicht-Arthritis, traumatische Arthritiden, Vulvodynie, Schädigungen und Erkrankungen der Muskeln und Faszien, juveniler Arthritis, Spondylitis, Psoriasis-Arthritis, Myositiden, Zahnerkrankungen, Influenza und anderen Virusinfektionen wie Erkältungen, systemischer Lupus erythematodes oder Schmerzen bei Verbrennungen;
(e) **Tumorschmerzen** im Zusammenhang mit Krebserkrankungen, wie beispielsweise lymphatischer oder myeloischer Leukämie, Hodgkin Erkrankung, Non-Hodgkin Lymphomen, Lymphogranulomatose, Lymphosarkomen, soliden malignen Tumoren und ausgedehnten Metastasen;
(f) **Kopfschmerz-Erkrankungen** unterschiedlicher Ursache, wie beispielsweise Cluster-Kopfschmerz, Migräne (mit oder ohne Aura) und Spannungskopfschmerz;
(g) Schmerzzuständen gemischter Ursache, wie beispielsweise chronische Rückenschmerzen, einschließlich Lumbago, oder Fibromyalgie.

Weiterhin eigenen sich die Verbindungen zur Behandlung von
(h) **entzündlichen und/oder ödematösen Erkrankungen der Haut und Schleimhäute**, wie beispielsweise allergische und nicht-allergische Dermatitiden, atopische Dermatitis, Psoriasis, Verbrennungen, Sonnenbrand, bakterielle Entzündungen, Irritationen und Entzündungen ausgelöst durch Chemikalien oder Naturstoffe (Pflanzen, Insekten, Insektenstiche), Juckreiz; von Zahnfleischentzündungen, Ödemen nach Traumata durch Verbrennungen, Angioödemen oder Uveitis;
(i) entzündlichen Veränderungen im Zusammenhang mit **Erkrankungen der Atemwege** und der Lunge wie Asthma bronchiale, einschließlich allergischem Asthma (atopisch und nicht-atopisch) sowie Bronchospasmen bei Anstrengung, beruflichbedingtem Asthma, viraler oder bakterieller Exazerbation einer bestehenden Asthma-Erkrankung und anderen nicht-allergisch bedingten asthmatischen Erkrankungen; von chronischer Bronchitis sowie chronisch obstruktiver Lungen-Erkrankung (COPD) einschließlich Lungenemphysem, virale oder bakterielle Exazerbation von chronischer Bronchitis oder chronisch obstruktiver Bronchitis, akutem Atemnotsyndrom des Erwachsenen (ARDS), Bronchitis, Lungenentzündung, allergischer Rhinitis (saisonal und ganzjährig), vasomotorischer Rhinitis und Staublungen-Erkrankungen wie Aluminose, Anthrakose, Asbestose, Chalikose, Siderose, Silikose, Tabakose, Byssinose, exogen allergische Alveolitiden, Lungenfibrose, Bronchiektasien, Lungenerkrankungen bei alpha1-Antritrypsinmangel und Husten;
(j) **entzündlichen Erkrankungen des Gastrointestinaltraktes** einschließlich Morbus Crohn und Colitis ulzerosa, Reizdarmsyndrom, Pankreatitis;
(k) **Diabetes Mellitus** und dessen Folgen (wie beispielsweise diabetische Vaskulopathie, diabetische Neuropathie, diabetische Retinopathie) und von diabetischen Symptomen bei Insulitis (beispielsweise Hyperglycämie, Diurese, Proteinurie und erhöhte renale Nitrit- und Kallikrein-Exkretion);
(I) **Sepsis und septischem Schock** nach bakteriellen Infektionen oder nach Trauma;
(m) **entzündlichen Erkrankungen der Gelenke und des Bindegewebes** wie vaskulären Bindegewebserkrankungen, Zerrungen und Frakturen, sowie muskuloskeletale Erkrankungen mit Entzündungserscheinungen wie akutes rheumatisches Fieber, Polymyalgia rheumatica, reaktive Arthritiden, Rheumatoide Arthritis, Spondylarthritiden, aber auch Osteoarthritis, sowie entzündliche Bindegewebserkrankungen anderer Genese, und Kollagenosen jeglicher Genese wie systemischer Lupus erythematodes, Sklerodermie, Polymyositis, dermatomyositis, Sjögren Syndrom, Morbus Still oder Felty Syndrom; sowie vaskuläre Erkrankungen wie Panarteriitis nodosa, Polyarthritis nodosa, Periarteriitis nodosa, Arteriitis temporalis, Wegnersche Granulomatose, Riesenzellartriitis, Arteriosklerose, Erythema nodosum;
(n) **Erkrankungen und Schädigungen des Zentralnervensystems,** wie beispielsweise Hirnödeme sowie Behandlung und Vorbeugung von psychiatrischen Erkrankungen, wie beispielsweise Depressionen, zur Behandlung und Vorbeugung von Epilepsie;
(o) Störungen der **Motilität oder Spasmen** von respiratorischen, genito-urinalen, gastro-intestinalen inklusive biliärer, oder vaskulären Strukturen und Organen; (p) post-operativem Fieber;
(q) zur Behandlung und Vorbeugung von kardiovaskulären Erkrankungen, wie beispielsweise Bluthochdruck und verwandte Erkrankungen;
(r) zur Behandlung und Vorbeugung von Arteriosklerose und verwandten Erkrankungen;
(s) zur Behandlung und Vorbeugung von **Krebs** und verwandten Erkrankungen;
(t) zur Behandlung und Vorbeugung von **Erkrankungen des Urogenitaltraktes,** wie beispielsweise Harninkontinenz und verwandten Erkrankungen, von benigner Prostata-Hyperplasie und hyperaktiver Blase, Nephritis, Zystitis (interstitielle Zystitis);
(u) zur Behandlung und Vorbeugung von **krankhaftem Übergewicht** und verwandten Erkrankungen.

Die Substanzen eignen sich zur kausalen Behandlung im Sinne einer Verlangsamung oder Unterbrechung des Voranschreitens chronisch progredienter Erkrankungen, insbesondere von Osteoarthritis, rheumatoider Arthritis und Spondylarthritiden.

Ein weiterer Gegenstand der vorliegenden Erfindung umfasst die Verwendung der erfindungsgemäßen Verbindungen der eingangs erwähnten allgemeinen Formel **I** zur Herstellung eines Arzneimittels für eine therapeutische Anwendung in den voranstehend genannten Indikationen.

Bevorzugt werden die erfindungsgemäßen Verbindungen der allgemeinen Formel **I** zur Behandlung von Osteoarthritis, rheumatoider Arthritis oder COPD eingesetzt.

Bevorzugt werden die erfindungsgemäßen Verbindungen der allgemeinen Formel **I** weiterhin zur Behandlung von entzündlichen Erkrankungen der Haut, wie beispielsweise allergische und nicht-allergische Dermatitiden, atopische Dermatitis, Psoriasis, Verbrennungen, Sonnenbrand, bakterielle Entzündungen, Irritationen und Entzündungen ausgelöst durch Chemikalien oder Naturstoffe (Pflanzen, Insekten, Insektenstiche), oder Juckreiz eingesetzt.

Unter dem Ausdruck "Behandlung" oder "Therapie" ist eine therapeutische Behandlung von Patienten mit manifester, akuter oder chronischer Indikation zu verstehen, wobei einerseits die symptomatische (palliative) Behandlung zur Linderung der Krankheitssymptome und andererseits die kausale oder kurative Behandlung der Indikation mit dem Ziel, den pathologischen Zustand zu beenden, den Schweregrad des pathologischen Zustands zu vermindern oder die Progression des pathologischen Zustands zu verzögern, abhängig von der Art oder Schwere der Indikation, eingeschlossen sind.

Ein weiterer Gegenstand der vorliegenden Erfindung ist die Verwendung einer Verbindung der allgemeinen Formel **I** zur Herstellung eines Arzneimittels zur akuten und prophylaktischen Behandlung von akuten Schmerzen, Eingeweideschmerzen, neuropathischen Schmerzen, entzündlichen / Schmerzrezeptor-vermittelten Schmerzen, Tumorschmerzen, Kopfschmerz-Erkrankungen und Schmerzzuständen gemischter Ursache sowie weiterer der voranstehend genannten Erkrankungen. Dabei ist die Verwendung dadurch gekennzeichnet, dass sie die Verabreichung einer wirksamen Menge einer Verbindung der allgemeinen Formel **I** oder eines physiologisch verträglichen Salzes davon an einen Patienten beinhaltet, der einer solchen Behandlung bedarf.

Unter dem Begriff "Patient" wird vorzugsweise ein Mensch verstanden.

Neben der Eignung als Humantherapeutika, sind diese Substanzen auch nützlich in der veterinärmedizinischen Therapie von Haustieren, exotischen Tieren und Nutztieren.

Ein weiterer Gegenstand der vorliegenden Erfindung sind Arzneimittel, enthaltend wenigstens eine erfindungsgemäße Verbindung der allgemeinen Formel **I** sowie gegebenenfalls geeignete Zusatzstoffe und/oder Hilfsstoffe und/oder gegebenenfalls weitere Wirkstoffe.

Die zur Erzielung einer schmerzstillenden Wirkung erforderliche Dosierung variiert in Abhängigkeit vom Gewicht des Patienten, von der Applikationsart, der Indikation und dem Schweregrad der Erkrankung. Üblicherweise beträgt die zu verabreichende Wirkstoffmenge bei intravenöser Gabe 0.01 bis 3 mg/kg Körpergewicht, vorzugsweise 0.1 bis 1 mg/kg, und bei oraler Gabe 0.1 bis 8 mg/kg Körpergewicht, vorzugsweise 0.5 bis 3 mg/kg, jeweils einmal bis dreimal täglich.

Die erfindungsgemäßen Arzneimittel enthalten neben mindestens einer erfindungsgemäßen Verbindung der allgemeinen Formel **I** gegebenenfalls geeignete inerter Zusatzstoffe und/oder Hilfsstoffe, wie beispielsweise Trägermaterialien, Füllstoffe, Lösungsmittel, Verdünnungsmittel, Farbstoffe und/oder Bindemittel. Sie können als flüssige Arzneiformen in Form von Injektionslösungen, Tropfen oder Säfte, als halbfeste Arzneiformen in Form von Granulaten, Tabletten, Pellets, Patches, Kapseln, Pflaster/Sprühpflaster oder Aerosolen verabreicht werden.

Die erfindungsgemäß hergestellten Verbindungen können intravenös, subkutan, intramuskulär, intrarektal, intranasal, durch Inhalation, transdermal oder oral, peroral, parenteral, intradermal, buccal, rektal oder topisch, zum Beispiel auf die Haut, die Schleimhäute oder in die Augen verabreicht werden, wobei zur Inhalation insbesondere Aerosolformulierungen geeignet sind. Sie können gegebenenfalls zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Granulate, Tropfen, Säfte, Sirupe, Pulver, Suspensionen, Lösungen, Dosieraerosole oder Zäpfchen eingearbeitet werden.

### KOMBINATIONEN

Zur Behandlung von Schmerzen kann es vorteilhaft sein, die erfindungsgemäßen Verbindungen mit belebenden Stoffen wie Koffein oder anderen schmerzlindernden Wirkstoffen zu kombinieren. Stehen zur Behandlung der Ursache der Schmerzen geeignete Wirkstoffe zur Verfügung, so können diese mit den erfindungsgemäßen Verbindungen kombiniert werden.

Für eine Kombinationstherapie kommen beispielsweise die folgenden Verbindungen in Frage:
Nicht-steroidale Antirheumatika (NSAR), wie beispielsweise Propionsäure-Derivate, die ausgewählt sein können aus der Gruppe bestehend aus Alminoprofen, Bucloxinsäure, Carprofen, Fenoprofen, Ibuprofen, Ketoprofen, Naproxen, Oxaprozin, Pirprofen, Pranoprofen und Tiaprofensäure; Essigsäure-Derivate, die ausgewählt sein können aus der Gruppe bestehend aus Indomethacin, Acemetacin, Alcofenac, Isoxepac, Sulindac und Tolmetin; Fenaminsäure-Derivate, die ausgewählt sein können aus der Gruppe bestehend aus Meclofenaminsäure, Mefenaminsäure und Tolfenaminsäure; Biphenyl-Carboxylsäure-Derivate; Oxicame, die ausgewählt sein können aus der Gruppe bestehend aus Meloxicam, Piroxicam und Tenoxicam; Salicylsäure-Derivate, die ausgewählt sein können aus der Gruppe bestehend aus Acetylsalicylsäure und Sulfasalazin; Pyrazolone, die ausgewählt sein können aus der Gruppe bestehend aus Apazon und Feprazon); Coxibe, die ausgewählt sein können aus der Gruppe bestehend aus Celecoxib und Etoricoxib.
Opiat Rezeptor Agonisten, die beispielsweise ausgewählt sein können aus der Gruppe bestehend aus wie Morphin, Darvon, Tramadol und Buprenorphin.
Cannabinoid Agonisten, wie beispielsweise GW-1000.
Natriumkanalblocker, die beispielsweise ausgewählt sein können aus der Gruppe bestehend aus Carbamazepin, Mexiletin, Pregabalin, Tectin und Ralfinamide.
N-Typ Calciumkanalblocker, wie beispielsweise Ziconotid.
Serotonerge und noradrenerge Modulatoren, die beispielsweise ausgewählt sein können aus der Gruppe bestehend aus Duloxetin und Amitriptylin.
Corticosteroide, die beispielsweise ausgewählt sein können aus der Gruppe bestehend aus Betamethason, Budesonid, Cortison, Dexamethason, Hydrocortison, Methylprednisolon, Prednisolon, Prednison und Triamcinolon.
Histamin H1-Rezeptorantagonisten, die beispielsweise ausgewählt sein können aus der Gruppe bestehend aus Brompheniramin, Chlorpheniramin, Dexchlorpheniramin, Triprolidin, Clemastin, Diphenhydramin, Diphenylpyralin, Tripelennamin, Hydroxyzin, Promethazin, Trimeprazin, Azatadin, Cyproheptadin, Antazolin, Pheniramin, Pyrilamin, Loratadin, Cetirizin, Desloratadin, Fexofenadin und Levocetirizin.
Leukotrien Antagonisten und 5-Lipoxygenasehemmer, die beispielsweise ausgewählt sein können aus der Gruppe bestehend aus Zafirlukast, Montelukast, Pranlukast und Zileuton.
Lokalanästhetika, die beispielsweise ausgewählt sein können aus der Gruppe bestehend aus Ambroxol und Lidocain.
TRPV1 Antagonisten, die beispielsweise ausgewählt sein können aus der Gruppe bestehend aus AZD-1386, JTS-653 und PHE-377.
Nikotinrezeptor Agonisten, wie beispielsweise A-366833.
P2X3-Rezeptor Antagonisten, wie beispielsweise A-317491.
anti-NGF Antikörper und NGF Antagonisten, die beispielsweise ausgewählt sein können aus der Gruppe bestehend aus JNJ-42160443 und PPH 207.
NK1 und NK2 Antagonisten, wie beispielsweise CP-728663.
NMDA Antagonisten, die beispielsweise ausgewählt sein können aus der Gruppe bestehend aus CNS-5161, AZ-756 und V-3381.
Kaliumkanal Modulatoren, wie beispielsweise CL-888.
GABA Modulatoren, wie beispielsweise Baclofen.
Anti-Migräne Therapeutika, die beispielsweise ausgewählt sein können aus der Gruppe bestehend aus Sumatriptan, Zolmitriptan, Naratriptan und Eletriptan.

Zur Behandlung von einer oder mehrerer der voranstehend genannten Atemwegserkrankungen kann es vorteilhaft sein, die erfindungsgemäßen Verbindungen der allgemeinen Formel **I** mit anderen Wirkstoffen zur Behandlung von Atemwegserkrankungen zu kombinieren. Stehen zur Behandlung der Ursache der Atemwegserkrankungen geeignete Wirkstoffe zur Verfügung, so können diese mit den erfindungsgemäßen Verbindungen kombiniert werden.

Gegebenenfalls können die Verbindungen der allgemeinen Formel **I** auch in Kombination mit weiteren pharmakologisch aktiven Wirkstoffen eingesetzt werden. Bevorzugt gelangen hierbei solche Wirkstoffe zur Anwendung, die beispielsweise ausgewählt sind aus der Gruppe bestehend aus Betamimetika, Anticholinergika, Corticosteroiden, PDE4-Inhibitoren, LTD4-Rezeptor-Antagonisten, Inhibitoren von MAP-Kinasen, EGFR-Hemmern, H1-Rezeptor Antagonisten, H4-Rezeptor-Antagonisten, PAF-Antagonisten, PI3-Kinase Inhibitoren, CXCR1 und/ oder CXCR2 Rezeptor-Antagonisten und Substanzen gegen Husten.

Als Betamimetika gelangen erfindungsgemäß vorzugsweise Verbindungen zur Anwendung, die ausgewählt sind aus der Gruppe bestehend aus Arformoterol, Carmoterol, Formoterol, Indacaterol, Salmeterol, Albuterole, Bambuterol, Bitolterol, Broxaterol, Carbuterol, Clenbuterol, Fenoterol, Hexoprenalin, Ibuterol, Isoetharin, Isoprenalin, Levosalbutamol, Mabuterol, Meluadrin, Metaproterenol, Milveterol, Orciprenalin, Pirbuterol, Procaterol, Reproterol, Rimiterol, Ritodrin, Salmefamol, Soterenol, Sulphonterol, Terbutalin, Tiaramid, Tolubuterol und Zinterol oder
- 6-Hydroxy-8-{1-hydroxy-2-[2-(4-methoxy-phenyl)-1,1-dimethyl-ethylamino]-ethyl}-4H-benzo[1,4]oxazin-3-on,
- 8-{2-[2-(2,4-Difluor-phenyl)-1,1-dimethyl-ethylamino]-1-hydroxy-ethyl}-6-hydroxy-4H-benzo[1,4]oxazin-3-on,
- 8-{2-[2-(3,5-Difluor-phenyl)-1,1-dimethyl-ethylamino]-1-hydroxy-ethyl}-6-hydroxy-4H-benzo[1,4]oxazin-3-on,
- 8-{2-[2-(4-Ethoxy-phenyl)-1,1-dimethyl-ethylamino]-1-hydroxy-ethyl}-6-hydroxy-4H-benzo[1,4]oxazin-3-on,
- 8-{2-[2-(4-Fluor-phenyl)-1,1-dimethyl-ethylamino]-1-hydroxy-ethyl}-6-hydroxy-4H-benzo[1,4]oxazin-3-on,
- N-(5-{2-[3-(4,4-Diethyl-2-oxo-4H-benzo[d][1,3]oxazin-1-yl)-1,1-dimethyl-propylamino]-1-hydroxy-ethyl}-2-hydroxy-phenyl)-methansulfonamid,
- N-(5-{2-[3-(4,4-Diethyl-6-fluoro-2-oxo-4H-benzo[d][1,3]oxazin-1-yl)-1,1-dimethyl-propylamino]-1-hydroxy-ethyl}-2-hydroxy-phenyl)-methansulfonamid,
- N-(5-{2-[3-(4,4-Diethyl-6-methoxy-2-oxo-4H-benzo[d][1,3]oxazin-1-yl)-1,1-dimethyl-propylamino]-1-hydroxy-ethyl}-2-hydroxy-phenyl)-methansulfonamid,
- N-(5-{2-[1,1-Dimethyl-3-(2-oxo-4,4-dipropyl-4H-benzo[d][1,3]oxazin-1-yl)-propylamino]-1-hydroxy-ethyl}-2-hydroxy-phenyl)-methansulfonamid,
- 8-{2-[1,1-Dimethyl-3-(2-oxo-2,3-dihydro-benzoimidazol-1-yl)-propylamino]-1-hydroxy-ethyl}-6-hydroxy-4H-benzo[1,4]oxazin-3-on,
- 8-{2-[1,1-Dimethyl-3-(6-methyl-2-oxo-2,3-dihydro-benzoimidazol-1-yl)-propylamino]-1-hydroxy-ethyl}-6-hydroxy-4H-benzo[1,4]oxazin-3-on,
- 8-{2-[1,1-Dimethyl-3-(2-oxo-5-trifluormethyl-2,3-dihydro-benzoimidazol-1-yl)-propylamino]-1-hydroxy-ethyl}-6-hydroxy-4H-benzo[1,4]oxazin-3-on,
- 8-{2-[1,1-Dimethyl-3-(3-methyl-2-oxo-2,3-dihydro-benzoimidazol-1-yl)-propylamino]-1-hydroxy-ethyl}-6-hydroxy-4H-benzo[1,4]oxazin-3-on,
- N-[2-Hydroxy-5-((1R)-1-hydroxy-2-{2-[4-(2-hydroxy-2-phenyl-ethylamino)-phenyl]-ethylamino}-ethyl)-phenyl]-formamid,
- 8-Hydroxy-5-((1R)-1-hydroxy-2-{2-[4-(6-methoxy-biphenyl-3-ylamino)-phenyl]-ethylamino}-ethyl)-1H-chinolin-2-on,
- 8-Hydroxy-5-[(1R)-1-hydroxy-2-(6-phenethylamino-hexylamino)-ethyl]-1H-chinolin-2-on,
- 5-[(1R)-2-(2-{4-[4-(2-Amino-2-methyl-propoxy)-phenylamino]-phenyl}-ethylamino)-1-hydroxy-ethyl]-8-hydroxy-1H-chinolin-2-on,
- [3-(4-{6-[(2R)-2-Hydroxy-2-(4-hydroxy-3-hydroxymethyl-phenyl)-ethylamino]-hexyloxy}-butyl)-5-methyl-phenyl]-harnstoff,
- 4-((1R)-2-{6-[2-(2,6-Dichlor-benzyloxy)-ethoxy]-hexylamino}-1-hydroxy-ethyl)-2-hydroxymethyl-phenol,
- 3-(4-{6-[(2R)-2-Hydroxy-2-(4-hydroxy-3-hydroxymethyl-phenyl)-ethylamino]-hexyloxy}-butyl)-benzenesulfonamid,
- 3-(3-{7-[(2R)-2-Hydroxy-2-(4-hydroxy-3-hydroxymethyl-phenyl)-ethylamino]-heptyloxy}-propyl)-benzenesulfonamid,
- 4-((1R)-2-{6-[4-(3-Cyclopentanesulfonyl-phenyl)-butoxy]-hexylamino}-1-hydroxy-ethyl)-2-hydroxymethyl-phenol,
- *N*-1-Adamantanyl-2-{3-[(2R)-2-({(2R)-2-hydroxy-2-[4-hydroxy-3-(hydroxymethyl)-phenyl]ethyl}amino)propyl]phenyl}acetamid,
- (1R)-5-{2-[6-(2,2-Difluor-2-phenyl-ethoxy)-hexylamino]-1-hydroxy-ethyl}-8-hydroxy-1 H-chinolin-2-on,
- (R,S)-4-(2-{[6-(2,2-Difluor-4-phenylbutoxy)hexyl]amino}-1-hydroxy-ethyl)-2-(hydroxyl-methyl)phenol,
- (R,S)-4-(2-{[6-(2,2-Difluor-2-phenylethoxy)hexyl]amino}-1-hydroxy-ethyl)-2-(hydroxyl-methyl)phenol,
- (R,S)-4-(2-{[4,4-Difluor-6-(4-phenylbutoxy)hexyl]amino}-1-hydroxy-ethyl)-2-(hydroxyl-methyl)phenol,
- (R,S)-4-(2-{[6-(4,4-Difluor-4-phenylbutoxy)hexyl]amino}-1-hydroxy-ethyl)-2-(hydroxyl-methyl)phenol,
- (R,S)-5-(2-{[6-(2,2-Difluor-2-phenylethoxy)hexyl]amino}-1-hydroxy-ethyl)-8-hydroxychinolin-2(1 H)-on,
- (R,S)-[2-({6-[2,2-Difluor-2-(3-methylphenyl)ethoxy]hexyl}amino)-1-hydroxyethyl]-2-(hydroxymethyl)phenol,
- 4-(1R)-2-{[6-(2,2-Difluor-2-phenylethoxy)hexyl]amino}-1-hydroxyethyl)-2-(hydroxyl-methyl)phenol,
- (R,S)-2-(Hydroxymethyl)-4-(1-hydroxy-2-{[4,4,l5-tetrafluor-6-(3-phenylpropoxy)-hexyl]amino}ethyl)phenol,
- (R,S)-[5-(2-[[6-(2,2-Difluor-2-phenylethoxy)hexyl]amino}-1-hydroxy-ethyl)-2-hydroxyphenyl]formamid,
- (R,S)-4-[2-({6-[2-(3-Bromophenyl)-2,2-difluoroethoxy]hexyl}amino)-1-hydroxyethyl]-2-(hydroxymethyl)phenol,
- (R, S)-N-[3-(1,1 -Difluor-2-{[6-({2-hydroxy-2-[4-hydroxy-3-(hydroxymethyl)phenyl]-ethyl}amino)hexyl]oxy}ethyl)phenyl]-harnstoff,
- 3-[3-(1,1-Difluor-2-{[6-({2-hydroxy-2-[4-hydroxy-3-(hydroxymethyl) phenyl]ethyl}-amino)hexyl]oxy}ethyl)phenyl]imidazolidin-2,4-dion,
- (R,S)-4-[2-({6-[2,2-Difluor-2-(3-methoxyphenyl)ethoxy]hexyl}amino)-1-hydroxyethyl]-2-(hydroxymethyl)phenol,
- 5-((1R)-2-{[6-(2,2-Difluor-2-phenylethoxy)hexyl]amino}-1-hydroxyethyl)-8-hydroxy-chinolin-2(1 H)-on,
- 4-((1R)-2-{[4,4-Difluor-6-(4-phenylbutoxy)hexyl]amino}-1-hydroxy-ethyl)-2-(hydroxyl-methyl)phenol,
- (R,S)-4-(2-{[6-(3,3-Difluor-3-phenylpropoxy)hexyl]amino}-1-hydroxy-ethyl)-2-(hydroxyl-methyl)phenol,
- (R,S)-(2-{[6-(2,2-Difluor-2-phenylethoxy)-4,4-difluorohexyl]amino}-1-hydroxyethyl)-2-(hydroxymethyl)phenol,
- (R,S)-4-(2-{[6-(2,2-Difluor-3-phenylpropoxy)hexyl]amino}-1-hydroxyethyl)-2-(hydroxyl-methyl)phenol,
- 3-[2-(3-Chlor-phenyl)-ethoxy]-N-(2-diethylamino-ethyl)-N-{2-[2-(4-hydroxy-2-oxo-2,3-dihydro-benzothiazol-7-yl)-ethylamino]-ethyl}-propionamid,
- N-(2-Diethylamino-ethyl)-N-{2-[2-(4-hydroxy-2-oxo-2,3-dihydro-benzothiazol-7-yl)-ethylamino]-ethyl}-3-(2-naphthalen-1-yl-ethoxy)-propionamid,
- 7-[2-(2-{3-[2-(2-Chlor-phenyl)-ethylamino]-propylsulfanyl}-ethylamino)-1-hydroxyethyl]-4-hydroxy-3H-benzothiazol-2-on,
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als **Anticholinergika** gelangen erfindungsgemäß vorzugsweise Verbindungen zur Anwendung, die ausgewählt sind aus der Gruppe bestehend aus Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Aclidiniumsalze, bevorzugt das Bromidsalz, Glycopyrroniumsalzen, bevorzugt das Bromidsalz, Trospiumsalzen, bevorzugt das Chloridsalz, Tolterodin, (3*R*)-1-Phenethyl-3-(9*H*-xanthen-9-carbonyloxy)-1-azoniabicyclo[2.2.2]octan-Salze. In den vorstehend genannten Salzen stellen die Kationen die pharmakologisch aktiven Bestandteile dar. Als Anionen X⁻ können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt. Weitere Anticholinergika können ausgewählt sein aus der Gruppe bestehend aus
- 2,2-Diphenylpropionsäuretropenolester-Methobromid,
- 2,2-Diphenylpropionsäurescopinester-Methobromid,
- 2-Fluor-2,2-Diphenylessigsäurescopinester-Methobromid,
- 2-Fluor-2,2-Diphenylessigsäuretropenolester-Methobromid,
- 3,3',4,4'-Tetrafluorbenzilsäuretropenolester-Methobromid,
- 3,3',4,4'-Tetrafluorbenzilsäurescopinester-Methobromid,
- 4,4'-Difluorbenzilsäuretropenolester-Methobromid,
- 4,4'-Difluorbenzilsäurescopinester-Methobromid,
- 3,3'-Difluorbenzilsäuretropenolester-Methobromid,
- 3,3'-Difluorbenzilsäurescopinester-Methobromid,
- 9-Hydroxy-fluoren-9-carbonsäuretropenolester-Methobromid,
- 9-Fluor-fluoren-9-carbonsäuretropenolester-Methobromid,
- 9-Hydroxy-fluoren-9-carbonsäurescopinester-Methobromid,
- 9-Fluor-fluoren-9-carbonsäurescopinester-Methobromid,
- 9-Methyl-fluoren-9-carbonsäuretropenolester-Methobromid,
- 9-Methyl-fluoren-9-carbonsäurescopinester-Methobromid,
- Benzilsäurecyclopropyltropinester-Methobromid,
- 2,2-Diphenylpropionsäurecyclopropyltropinester-Methobromid,
- 9-Hydroxy-xanthen-9-carbonsäurecyclopropyltropinester-Methobromid,
- 9-Methyl-fluoren-9-carbonsäurecyclopropyltropinester-Methobromid,
- 9-Methyl-xanthen-9-carbonsäurecyclopropyltropinester-Methobromid,
- 9-Hydroxy-fluoren-9-carbonsäurecyclopropyltropinester-Methobromid,
- 4,4'-Difluorbenzilsäuremethylestercyclopropyltropinester-Methobromid,
- 9-Hydroxy-xanthen-9-carbonsäuretropenolester-Methobromid,
- 9-Hydroxy-xanthen-9-carbonsäurescopinester-Methobromid,
- 9-Methyl-xanthen-9-carbonsäuretropenolester-Methobromid,
- 9-Methyl-xanthen-9-carbonsäurescopinester-Methobromid,
- 9-Ethyl-xanthen-9-carbonsäuretropenolester-Methobromid,
- 9-Difluormethyl-xanthen-9-carbonsäuretropenolester-Methobromid, und
- 9-Hydroxymethyl-xanthen-9-carbonsäurescopinester-Methobromid.

Als **Corticosteroide** gelangen erfindungsgemäß vorzugsweise Verbindungen zur Anwendung, die ausgewählt sind aus der Gruppe bestehend aus Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon und Tipredane oder
- Pregna-1,4-dien-3,20-dion, 6-Fluor-11-hydroxy-16,17-[(1-methylethyliden)-bis(oxy)]-21-[[4-[(nitrooxy)methyl]benzoyl]oxy]-, (6-alpha, 11-beta,16-alpha)-(9Cl) (NCX-1024)
- 16,17-Butylidendioxy-6,9-difluor-11-hydroxy-17-(methylthio)androst-4-en-3-on (RPR-106541),
- 6,9-Difluor-17-[(2-furanylcarbonyl)oxy]-11-hydroxy-16-methyl-3-oxo-androsta-1,4-dien-17-carbothionsäure-(S)-fluormethylester,
- 6,9-Difluor-11-hydroxy-16-methyl-3-oxo-17-propionyloxy-androsta-1,4-dien-17-carbo-thionsäure (S)-(2-oxo-tetrahydrofuran-3S-yl)ester, und
- 6-alpha,9-alpha-Difluor-11-beta-hydroxy-16alpha-methyl-3-oxo-17alpha-(2,2,3,3-tetramethylcyclopropylcarbonyl)oxy-androsta-1,4-dien-17beta-carbonsäure-cyanomethylester,
gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als **PDE4-Inhibitoren** gelangen erfindungsgemäß vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Apremilast, Arofyllin, Atizoram, Oglemilast und Tetomilast oder
- 5-[(N-(2,5-Dichlor-3-pyridinyl)-carboxamid]-8-methoxy-chinolin (D-4418),
- 5-[N-(3,5-Dichlor-1-oxido-4-pyridinyl)-carboxamid]-8-methoxy-2-(trifluormethyl)-chinolin (D-4396 (Sch-351591)),
- N-(3,5-Dichlorpyrid-4-yl)-[1-(4-fluorbenzyl)-5-hydroxy-indol-3-yl]glyoxylsäureamid (AWD-12-281 (GW-842470)),
- 9-[(2-Fluorphenyl)methyl]-N-methyl-2-(trifluormethyl)-9H-purin-6-amin (NCS-613),
- 4-[(2R)-2-[3-(Cyclopentyloxy)-4-methoxyphenyl]-2-phenylethyl]-pyridin (CDP-840),
- N-[(3R)-3,4,6,7-Tetrahydro-9-methyl-4-oxo-1-phenylpyrrolo[3,2,1-jk][1,4]benzo-diazepin-3-yl]-4-pyridincarboxamid (PD-168787),
- 4-[6,7-Diethoxy-2,3-bis(hydroxymethyl)-1-naphthalenyl]-1-(2-methoxyethyl)-2(1H)-pyridinon (T-440),
- 2-[4-[6,7-Diethoxy-2,3-bis(hydroxymethyl)-1-naphthalenyl]-2-pyridinyl]-4-(3-pyridinyl)-1(2H)-phthalazinon (T-2585),
- (3-(3-Cyclopenyloxy-4-methoxybenzyl)-6-ethylamino-8-isopropyl-3H-purin (V-11294A),
- beta-[3-(Cyclopentyloxy)-4-methoxyphenyl]-1,3-dihydro-1,3-dioxo-2H-isoindol-2-propanamid (CDC-801),
- Imidazo[1,5-a]pyrido[3,2-e]pyrazin-6(5H)-on, 9-ethyl-2-methoxy-7-methyl-5-propyl-(D-22888),
- 5-[3-(cyclopentyloxy)-4-methoxyphenyl]-3-[(3-methylphenyl)methyl]-, (3S,5S)-2-Piperi-dinon (HT-0712),
- 4-[1-[3,4-bis(difluoromethoxy)phenyl]-2-(3-methyl-1-oxido-4-pyridinyl)ethyl]-alpha,alpha-bis(trifluoromethyl)-Benzenemethanol (L-826141),
- N-(3,5-Dichlor-1-oxo-pyridin-4-yl)-4-difluormethoxy-3-cyclopropylmethoxybenzamid,
- (-)p-[(4aR*,10*b*S*)-9-Ethoxy-1,2,3,4,4a,10b-hexahydro-8-methoxy-2-methylbenzo[s]-[1,6]naphthyridin-6-yl]-N,N-diisopropylbenzamid,
- (R)-(+)-1-(4-Brombenzyl)-4-[(3-cyclopentyloxy)-4-methoxyphenyl]-2-pyrrolidon,
- 3-(Cyclopentyloxy-4-methoxyphenyl)-1-(4-N'-[N-2-cyano-S-methyl-isothioureido]-benzyl)-2-pyrrolidon,
- cis[4-Cyano-4-(3-cyclopentyloxy-4-methoxyphenyl)cyclohexan-1-carbonsäure],
- 2-Carbomethoxy-4-cyano-4-(3-cyclopropylmethoxy-4-difluoromethoxyphenyl)-cyclohexan-1-on,
- cis[4-Cyano-4-(3-cyclopropylmethoxy-4-difluormethoxyphenyl)cyclohexan-1-ol],
- (R)-(+)-Ethyl[4-(3-cyclopentyloxy-4-methoxyphenyl)pyrrolidin-2-yliden]acetate,
- (S)-(-)-Ethyl[4-(3-cyclopentyloxy-4-methoxyphenyl)pyrrolidin-2-yliden]acetate,
- 9-Cyclopentyl-5,6-dihydro-7-ethyl-3-(2-thienyl)-9*H*-pyrazolo[3,4-c]-1,2,4-triazolo-[4,3-a]pyridin und
- 9-Cyclopentyl-5,6-dihydro-7-ethyl-3-(*tert*-butyl)-9*H*-pyrazolo[3,4-c]-1,2,4-triazolo-[4,3-a]pyridin,
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als **LTD4-Rezeptor Antagonisten** gelangen erfindungsgemäß vorzugsweise Verbindungen zur Anwendung, die ausgewählt sind aus der Gruppe bestehend aus Montelukast, Pranlukast und Zafirlukast, oder
- (E)-8-[2-[4-[4-(4-Fluorphenyl)butoxy]phenyl]ethenyl]-2-(1H-tetrazol-5-yl)-4H-1-benzopyran-4-on (MEN-91507),
- 4-[6-Acetyl-3-[3-(4-acetyl-3-hydroxy-2-propylphenylthio)propoxy]-2-propylphenoxy]-buttersäure (MN-001),
- 1-(((R)-(3-(2-(6,7-Difluor-2-chinolinyl)ethenyl)phenyl)-3-(2-(2-hydroxy-2-propyl)phenyl)-thio)methylcyclopropan-essigsäure,
- 1-(((1(R)-3(3-(2-(2,3-Dichlorthieno[3,2-b]pyridin-5-yl)-(E)-ethenyl)phenyl)-3-(2-(1-hydroxy-1-methylethyl)phenyl)propyl)thio)methyl)cyclopropanessigsäure und
- [2-[[2-(4-tert-Butyl-2-thiazolyl)-5-benzofuranyl]oxymethyl]phenyl]essigsäure,
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Rezeptor Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als **MAP Kinase Inhibitoren** gelangen erfindungsgemäß vorzugsweise Verbindungen zur Anwendung, die ausgewählt sind aus der Gruppe bestehend aus:
- Bentamapimod (AS-602801)
- Doramapimod,
- 5-Carbamoylindole (SD-169),
- 6-[(Aminocarbonyl)(2,6-difluorphenyl)amino]-2-(2,4-difluorphenyl)-3-pyridincarboxamid (VX-702),
- alpha-[2-[[2-(3-Pyridinyl)ethyl]amino]-4-pyrimidinyl]-2-benzothiazoleacetonitril (AS-601245),
- 9,12-Epoxy-1H-diindolo[1,2,3-fg:3',2',1'-kl]pyrrolo[3,4-i][1,6]benzodiazocin-10-Carboxylsäure (CEP-1347), und
- 4-[3-(4-Chlorphenyl)-5-(1-methyl-4-piperidinyl)-1 H-pyrazol-4-yl]-pyrimidin (SC-409),
gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als **EGFR-Hemmer** gelangen erfindungsgemäß vorzugsweise Verbindungen zur Anwendung, die ausgewählt sind aus der Gruppe bestehend aus Cetuximab, Trastuzumab, Panitumumab (= ABX-EGF), Mab ICR-62, Gefitinib, Canertinib und Erlotinib oder
- 4-[(3-Chlor-4-fluorphenyl)amino]-6-{[4-(morpholin-4-yl)-1-oxo-2-buten-1-yl]amino}-7-cyclopropylmethoxy-chinazolin,
- 4-[(3-Chlor-4-fluorphenyl)amino]-6-{[4-(N,N-diethylamino)-1-oxo-2-buten-1-yl]amino}-7-cyclopropylmethoxy-chinazolin,
- 4-[(3-Chlor-4-fluorphenyl)amino]-6-{[4-(N,N-dimethylamino)-1-oxo-2-buten-1-yl]amino}-7-cyclopropylmethoxy-chinazolin,
- 4-[(R)-(1-Phenyl-ethyl)amino]-6-{[4-(morpholin-4-yl)-1-oxo-2-buten-1-yl]amino}-7-cyclopentyloxy-chinazolin,
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin,
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin,
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin,
- 4-[(3-Chlor-4-fluor-phenyl)amino]-6-[2-((S)-6-methyl-2-oxo-morpholin-4-yl)-ethoxy]-7-methoxy-chinazolin,
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin,
- 4-[(3-Chlor-4-fluorphenyl)amino]-6-{[4-(N,N-dimethylamino)-1-oxo-2-buten-1-yl]amino}-7-cyclopentyloxy-chinazolin,
- 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin,
- 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin,
- 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin,
- 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin,
- 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin,
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin,
- 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin,
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin,
- 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin,
- 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin,
- 4-[(3-Ethinyl-phenyl)amino]-6,7-bis-(2-methoxy-ethoxy)-chinazolin,
- 4-[(3-Chlor-4-fluorphenyl)amino]-7-[3-(morpholin-4-yl)-propyloxy]-6-[(vinylcarbonyl)-amino]-chinazolin,
- 4-[(R)-(1-Phenyl-ethyl)amino]-6-(4-hydroxy-phenyl)-7H-pyrrolo[2,3-d]pyrimidin,
- 3-Cyano-4-[(3-chlor-4-fluorphenyl)amino]-6-{[4-(N,N-dimethylamino)-1-oxo-2-buten-1-yl]amino}-7-ethoxy-chinolin,
- 4-{[3-Chlor-4-(3-fluor-benzyloxy)-phenyl]amino}-6-(5-{[(2-methansulfonyl-ethyl)amino]-methyl}-furan-2-yl)chinazolin,
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin,
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin,
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin,
- 4-[(3-Ethinyl-phenyl)amino]-6-{[4-(5,5-dimethyl-2-oxo-morpholin-4-yl)-1-oxo-2-buten-1-yl]amino}-chinazolin,
- 4-[(3-Chlor-4-fluor-phenyl)amino]-6-[2-(2,2-dimethyl-6-oxo-morpholin-4-yl)-ethoxy]-7-methoxy-chinazolin,
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin,
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin,
- 4-[(3-Chlor-4-fluor-phenyl)amino]-6-{2-[4-(2-oxo-morpholin-4-yl)-piperidin-1-yl]-ethoxy}-7-methoxy-chinazolin,
- 4-[(3-Chlor-4-fluor-phenyl)amino]-6-[1-(*tert*-butyloxycarbonyl)-piperidin-4-yloxy]-7-methoxy-chinazolin,
- 4-[(3-Chlor-4-fluor-phenyl)amino]-6-(trans-4-amino-cyclohexan-1-yloxy)-7-methoxy-chinazolin,
- 4-[(3-Chlor-4-fluor-phenyl)amino]-6-(trans-4-methansulfonylamino-cyclohexan-1-yloxy)-7-methoxy-chinazolin,
- 4-[(3-Chlor-4-fluor-phenyl)amino]-6-(tetrahydropyran-3-yloxy)-7-methoxy-chinazolin,
- 4-[(3-Chlor-4-fluor-phenyl)amino]-6-(1-methyl-piperidin-4-yloxy)-7-methoxy-chinazolin,
- 4-[(3-Chlor-4-fluor-phenyl)amino]-6-{1-[(morpholin-4-yl)carbonyl]-piperidin-4-yloxy}-7-methoxy-chinazolin,
- 4-[(3-Chlor-4-fluor-phenyl)amino]-6-{1-[(methoxymethyl)carbonyl]-piperidin-4-yloxy}-7-methoxy-chinazolin,
- 4-[(3-Chlor-4-fluor-phenyl)amino]-6-(piperidin-3-yloxy)-7-methoxy-chinazolin,
- 4-[(3-Chlor-4-fluor-phenyl)amino]-6-[1-(2-acetylamino-ethyl)-piperidin-4-yloxy]-7-methoxy-chinazolin,
- 4-[(3-Chlor-4-fluor-phenyl)amino]-6-(tetrahydropyran-4-yloxy)-7-ethoxy-chinazolin,
- 4-[(3-Chlor-4-fluor-phenyl)amino]-6-((S)-tetrahydrofuran-3-yloxy)-7-hydroxy-chinazolin,
- 4-[(3-Chlor-4-fluor-phenyl)amino]-6-(tetrahydropyran-4-yloxy)-7-(2-methoxy-ethoxy)-chinazolin,
- 4-[(3-Chlor-4-fluor-phenyl)amino]-6-{trans-4-[(dimethylamino)sulfonylamino]-cyclohexan-1-yloxy}-7-methoxy-chinazolin,
- 4-[(3-Chlor-4-fluor-phenyl)amino]-6-{trans-4-[(morpholin-4-yl)carbonylamino]-cyclohexan-1-yloxy}-7-methoxy-chinazolin,
- 4-[(3-Chlor-4-fluor-phenyl)amino]-6-{trans-4-[(morpholin-4-yl)sulfonylamino]-cyclohexan-1-yloxy}-7-methoxy-chinazolin,
- 4-[(3-Chlor-4-fluor-phenyl)amino]-6-(tetrahydropyran-4-yloxy)-7-(2-acetylamino-ethoxy)-chinazolin,
- 4-[(3-Chlor-4-fluor-phenyl)amino]-6-(tetrahydropyran-4-yloxy)-7-(2-methansulfonyl-amino-ethoxy)-chinazolin,
- 4-[(3-Chlor-4-fluor-phenyl)amino]-6-{1-[(piperidin-1-yl)carbonyl]-piperidin-4-yloxy}-7-methoxy-chinazolin,
- 4-[(3-Chlor-4-fluor-phenyl)amino]-6-(1-aminocarbonylmethyl-piperidin-4-yloxy)-7-methoxy-chinazolin,
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin,
- 4-[(3-Chlor-4-f luor-phenyl)am i no]-6-(cis-4-{N-[(morpholin-4-yl)carbonyl]- N-methyl-amino}-cyclohexan-1-yloxy)-7-methoxy-chinazolin,
- 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazolin,
- 4-[(3-Chlor-4-fluor-phenyl)amino]-6-(trans-4-ethansulfonylamino-cyclohexan-1-yloxy)-7-methoxy-chinazolin,
- 4-[(3-Chlor-4-fluor-phenyl)amino]-6-(1-methansulfonyl-piperidin-4-yloxy)-7-ethoxy-chinazolin,
- 4-[(3-Chlor-4-fluor-phenyl)amino]-6-(1-methansulfonyl-piperidin-4-yloxy)-7-(2-methoxy-ethoxy)-chinazolin,
- 4-[(3-Chlor-4-fluor-phenyl)amino]-6-[1-(2-methoxy-acetyl)-piperidin-4-yloxy]-7-(2-methoxy-ethoxy)-chinazolin,
- 4-[(3-Chlor-4-fluor-phenyl)amino]-6-(cis-4-acetylamino-cyclohexan-1-yloxy)-7-methoxy-chinazolin,
- 4-[(3-Ethinyl-phenyl)amino]-6-[1-(*tert*-butyloxycarbonyl)-piperidin-4-yloxy]-7-methoxy-chinazolin,
- 4-[(3-Ethinyl-phenyl)amino]-6-(tetrahydropyran-4-yloxy]-7-methoxy-chinazolin,
- 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin,
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin,
- 4-[(3-Chlor-4-fluor-phenyl)amino]-6-{cis-4-[(morpholin-4-yl)carbonylamino]-cyclohexan-1-yloxy}-7-methoxy-chinazolin,
- 4-[(3-Chlor-4-fluor-phenyl)amino]-6-{1-[2-(2-oxopyrrolidin-1-yl)ethyl]-piperidin-4-yloxy}-7-methoxy-chinazolin,
- 4-[(3-Chlor-4-fluor-phenyl)amino]-6-{1-[(morpholin-4-yl)carbonyl]-piperidin-4-yloxy}-7-(2-methoxy-ethoxy)-chinazolin,
- 4-[(3-Ethinyl-phenyl)amino]-6-(1-acetyl-piperidin-4-yloxy)-7-methoxy-chinazolin,
- 4-[(3-Ethinyl-phenyl)amino]-6-(1-methyl-piperidin-4-yloxy)-7-methoxy-chinazolin,
- 4-[(3-Ethinyl-phenyl)amino]-6-(1-methansulfonyl-piperidin-4-yloxy)-7-methoxy-chinazolin,
- 4-[(3-Chlor-4-fluor-phenyl)amino]-6-(1-methyl-piperidin-4-yloxy)-7(2-methoxy-ethoxy)-chinazolin,
- 4-[(3-Chlor-4-fluor-phenyl)amino]-6-(1-isopropyloxycarbonyl-piperidin-4-yloxy)-7-methoxy-chinazolin,
- 4-[(3-Chlor-4-fluor-phenyl)amino]-6-(cis-4-methylamino-cyclohexan-1-yloxy)-7-methoxy-chinazolin,
- 4-[(3-Chlor-4-fluor-phenyl)amino]-6-{cis-4-[N-(2-methoxy-acetyl)-N-methyl-amino]-cyclohexan-1-yloxy}-7-methoxy-chinazolin,
- 4-[(3-Ethinyl-phenyl)amino]-6-(piperidin-4-yloxy)-7-methoxy-chinazolin,
- 4-[(3-Ethinyl-phenyl)amino]-6-[1-(2-methoxy-acetyl)-piperidin-4-yloxy]-7-methoxy-chinazolin,
- 4-[(3-Ethinyl-phenyl)amino]-6-{1-[(morpholin-4-yl)carbonyl]-piperidin-4-yloxy}-7-methoxy-chinazolin,
- 4-[(3-Chlor-4-fluor-phenyl)amino]-6-{1-[(cis-2,6-dimethyl-morpholin-4-yl)carbonyl]-piperidin-4-yloxy}-7-methoxy-chinazolin,
- 4-[(3-Chlor-4-fluor-phenyl)amino]-6-{1-[(2-methyl-morpholin-4-yl)carbonyl]-piperidin-4-yloxy}-7-methoxy-chinazolin,
- 4-[(3-Chlor-4-fluor-phenyl)amino]-6-{1-[(S,S)-(2-oxa-5-aza-bicyclo[2.2.1]hept-5-yl)-carbonyl]-piperidin-4-yloxy}-7-methoxy-chinazolin,
- 4-[(3-Chlor-4-fluor-phenyl)amino]-6-{1-[(N-methyl-N-2-methoxyethyl-amino)carbonyl]-piperidin-4-yloxy}-7-methoxy-chinazolin,
- 4-[(3-Chlor-4-fluor-phenyl)amino]-6-(1-ethyl-piperidin-4-yloxy)-7-methoxy-chinazolin,
- 4-[(3-Chlor-4-fluor-phenyl)amino]-6-{1-[(2-methoxyethyl)carbonyl]-piperidin-4-yloxy}-7-methoxy-chinazolin,
- 4-[(3-Chlor-4-fluor-phenyl)amino]-6-{1-[(3-methoxypropyl-amino)-carbonyl]-piperidin-4-yloxy}-7-methoxy-chinazolin,
- 4-[(3-Chlor-4-fluor-phenyl)amino]-6-[cis-4-(N-methansulfonyl-N-methyl-amino)-cyclohexan-1-yloxy]-7-methoxy-chinazolin,
- 4-[(3-Chlor-4-fluor-phenyl)amino]-6-[cis-4-(N-acetyl-N-methyl-amino)-cyclohexan-1-yloxy]-7-methoxy-chinazolin,
- 4-[(3-Chlor-4-fluor-phenyl)amino]-6-(trans-4-methylamino-cyclohexan-1-yloxy)-7-methoxy-chinazolin,
- 4-[(3-Chlor-4-fluor-phenyl)amino]-6-[trans-4-(N-methansulfonyl-N-methyl-amino)-cyclohexan-1-yloxy]-7-methoxy-chinazolin,
- 4-[(3-Chlor-4-fluor-phenyl)amino]-6-(trans-4-dimethylamino-cyclohexan-1-yloxy)-7-methoxy-chinazolin,
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin,
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin,
- 4-[(3-Chlor-4-fluor-phenyl)amino]-6-(1-methansulfonyl-piperidin-4-yloxy)-7-methoxy-chinazolin,
- 4-[(3-Chlor-4-fluor-phenyl)amino]-6-(1-cyano-piperidin-4-yloxy)-7-methoxy-chinazolin,
- 3-Cyano-4-[(3-chlor-4-fluorphenyl)amino]-6-{[4-(N, N-dimethylamino)-1-oxo-2-buten-1-yl]amino}-7-ethoxy-chinolin ;
- [4-[(3-Chlor-4-fluorphenyl)amino]-6-{[4-(homomorpholin-4-yl)-1-oxo-2-buten-1-yl]-amino}-7-[(S)-(tetrahydrofuran-3-yl)oxy]-chinazolin,
- 4-[(3-Chlor-4-fluorphenyl)amino]-7-(2-{4-[(S)-(2-oxo-tetrahydrofuran-5-yl)carbonyl]-piperazin-1-yl}-ethoxy)-6-[(vinylcarbonyl)amino]-chinazolin,
- 4-[(3-Chlor-4-fluorphenyl)amino]-7-[2-((S)-6-methyl-2-oxo-morpholin-4-yl)-ethoxy]-6-[(vinylcarbonyl)amino]-chinazolin,
- 4-[(3-Chlor-4-fluorphenyl)amino]-7-[4-((R)-6-methyl-2-oxo-morpholin-4-yl)-butyloxy]-6-[(vinylcarbonyl)amino]-chinazolin,
- 4-[(3-Chlor-4-fluorphenyl)amino]-7-[4-((S)-6-methyl-2-oxo-morpholin-4-yl)-butyloxy]-6-[(vinylcarbonyl)amino]-chinazolin, und
- 4-[(3-Chlor-4-fluorphenyl)amino]-6-[(4-{N-[2-(ethoxycarbonyl)-ethyl]-N-[(ethoxy-carbonyl)methyl]amino}-1-oxo-2-buten-1-yl)amino]-7-cyclopropylmethoxy-chinazolin,
gegebenenfalls in Form ihrer Racemate, Enantiomere oder Diastereomere, gegebenenfalls in Form ihrer pharmakologisch verträglichen Säureadditionssalze, ihrer Solvate und/oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als **Histamin H1 Rezeptor Antagonisten** gelangen erfindungsgemäß vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Olopatadine, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als **Histamin H4 Rezeptor Antagonisten** gelangen erfindungsgemäß vorzugsweise Verbindungen zur Anwendung wie beispielsweise (5-Chlor-1 H-indol-2-yl)-(4-methyl-1-piperazinyl)-Methanon (JNJ-7777120), gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat

Als **PAF-Antagonisten** gelangen erfindungsgemäß vorzugsweise Verbindungen zur Anwendung, die ausgewählt sind aus der Gruppe bestehend aus Lexipafant und den Verbindungen
- 4-(2-Chlorphenyl)-9-methyl-2-[3(4-morpholinyl)-3-propanon-1-yl]-6H-thieno-[3,2-f]-[1,2,4]triazolo[4,3-a][1,4]diazepin und
- 6-(2-Chlorphenyl)-8,9-dihydro-1-methyl-8-[(4-morpholinyl)carbonyl]-4H,7H-cyclo-penta-[4,5]thieno-[3,2-f][1,2,4]triazolo[4,3-a][1,4]diazepin,
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als **PI3-Kinase Inhibitoren** gelangen erfindungsgemäß vorzugsweise Verbindungen zur Anwendung, die ausgewählt sind aus der Gruppe bestehend aus
- 5-(Quinoxalin-6-ylmethylene)thiazolidine-2,4-dione (AS-605240),
- 2-[(6-amino-9H-purin-9-yl)methyl]-5-methyl-3-(2-methylphenyl)-4(3H)-Quinazolinone (C-87114) und
- 2-Methyl-2-[4-[3-methyl-2-oxo-8-(chinolin-3-yl)-2,3-dihydroimidazo[4,5-c]chinolin-1-yl]phenyl]propionitrile (BEZ-235),
gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als **CXCR1 oder CXCR2 Antagonisten** gelangen erfindungsgemäß vorzugsweise Verbindungen zur Anwendung, die ausgewählt sind aus der Gruppe bestehend aus 3-[[3-[(Dimethylamino)carbonyl]-2-hydroxyphenyl]amino]-4-[[(R)-1-(5-methylfuran-2-yl)propyl]-amino]cyclobut-3-en-1,2-dion (SCH-527123), gegebenenfalls in Form seiner Racemate, Enantiomere, Diastereomere und gegebenenfalls in Form seiner pharmakologisch verträglichen Säureadditionssalze, Prodrugs, Solvate oder Hydrate.

Als **Substanzen gegen Husten** gelangen erfindungsgemäß vorzugsweise Verbindungen zur Anwendung, die ausgewählt sind aus der Gruppe bestehend aus Hydrocodone, Caramiphen, Carbetapentane und Dextramethorphan, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Zur Behandlung von **entzündlichen und/oder ödematösen Erkrankungen der Haut und Schleimhäute** können die erfindungsgemäßen Verbindungen der allgemeinen Formel I beispielsweise mit Substanzen kombiniert werden, die ausgewählt sind aus der Gruppe bestehend aus Methotrexat, Cyclosporin, topischen Steroiden, topische Calcineurin Inhibitoren, Vitamin-D-Analoga, Fumuraten, PDE4-Inhibitoren und TNF-Antagonisten, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Calcineurin Inhibitoren gelangen erfindungsgemäß vorzugsweise Verbindungen zur Anwendung, die ausgewählt sind aus der Gruppe bestehend aus Tacrolimus und Pimecrolimus.

Als Vitamin-D-Analogon gelangt erfindungsgemäß bevorzugt Calcipotriol zur Anwendung.

Als Fumurat gelangt erfindungsgemäß vorzugsweise BG 12 (orales Fumurat) zur Anwendung.

Als TNF-Antagonisten gelangen erfindungsgemäß vorzugsweise Verbindungen zur Anwendung, die ausgewählt sind aus der Gruppe bestehend aus Etanercept (Enbrel), Infliximab (Remicade) und Adalimumab (Humira).

Die zur Erzielung einer schmerzstillenden Wirkung erforderliche Dosierung beträgt bei intravenöser Gabe zweckmäßigerweise 0.01 bis 3 mg/kg Körpergewicht, vorzugsweise 0.1 bis 1 mg/kg, und bei oraler Gabe 0.1 bis 8 mg/kg Körpergewicht, vorzugsweise 0.5 bis 3 mg/kg, jeweils einmal, zweimal oder dreimal täglich. Die erfindungsgemäß hergestellten Verbindungen können intravenös, subkutan, intramuskulär, intrarektal, intranasal, durch Inhalation, transdermal oder oral verabreicht werden, wobei zur Inhalation insbesondere Aerosolformulierungen geeignet sind. Sie können gegebenenfalls zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen, Lösungen, Dosieraerosole oder Zäpfchen eingearbeitet werden.

### EXPERIMENTELLER TEIL

Für die hergestellten Verbindungen liegen in der Regel Massenspektren und/oder ¹H-NMR-Spektren vor. Die bei den Fliessmitteln angegebenen Verhältnisse beziehen sich auf Volumeneinheiten der jeweiligen Lösungsmittel. Die angegebenen Volumeneinheiten bei Ammoniak beziehen sich auf eine konzentrierte Lösung von Ammoniak in Wasser. Soweit nicht anders vermerkt sind die bei den Aufarbeitungen der Reaktionslösungen verwendeten Säure-, Basen- und Salzlösungen wässrige Systeme der angegebenen Konzentrationen.
Zu chromatographischen Reinigungen wird Kieselgel der Firma Millipore (MATREX*^{™}*, 35 bis 70 µm) oder Alox (E. Merck, Darmstadt, Aluminiumoxid 90 standardisiert, 63 bis 200 µm, Artikel-Nr: 1.01097.9050) verwendet.

In den Versuchsbeschreibungen werden die folgenden Abkürzungen verwendet:
- DC: Dünnschichtchromatogramm
- DIPEA: Diisopropylethylamin
- DMA: *N,N*-Dimethylacetamid
- DMAP: 4-Dimethylaminopyridin
- DMF: *N,N*-Dimethylformamid
- DMSO: Dimethylsulfoxid
- HATU: *O*-(7-Azabenzotriazol-1-yl)-*N,N,N',N'*-tetramethyluroniumhexafluorphosphat
- Pd₂(dba)₃: Tris(dibenzylideneacetone)-dipalladium(0)
- RP: Reverse Phase
- Rₜ: Retentionszeit
- *tert*: tertiär
- TBTU: 2-(1*H*-Benzotriazol-1-yl)-1,1,3,3-tetramethyluronium-tetrafluorborat
- TEA: Triethylamin
- THF: Tetrahydrofuran
- XPhos: 2-Dicyclohexyl-phosphino-2',4',6'-tri-isopropyl-1,1'-biphenyl

Folgende analytische HPLC-Methoden wurden verwendet:

### Methode 1:

Säule: Merck Cromolith Flash RP18e, 4.6 x 25 mm
Fließmittel A: Wasser / 0.1 % Ameisensäure
Fließmittel B: Acetonitril / 0.1 % Ameisensäure
Gradient:

| Zeit in min | %A | %B | Flussrate in mL/min |
|---|---|---|---|
| 0.0 | 90.0 | 10.0 | 1.6 |
| 2.7 | 10.0 | 90.0 | 1.6 |
| 3.0 | 10.0 | 90.0 | 1.6 |
| 3.3 | 90.0 | 10.0 | 1.6 |

### Methode 2:

- Säule:: Waters, Sunfire C18, 4.6 x 30 mm; 3.5 µm
- Fließmittel A:: Wasser / 0.1 % Trifluoressigsäure
- Fließmittel B:: Methanol / 0.1 % Trifluoressigsäure

Temperatur: 60°C
Gradient:

| Zeit in min | %A | %B | Flussrate in mL/min |
|---|---|---|---|
| 0.0 | 95 | 5 | 4.0 |
| 0.15 | 95 | 5 | 4.0 |
| 1.7 | 0 | 100 | 4.0 |
| 2.25 | 0 | 100 | 4.0 |

### Methode 3:

Säule: Waters, Xbridge, C18, 4.6 x 30 mm; 3.5 µm
Fließmittel A: Wasser / 0.1 % Trifluoressigsäure
Fließmittel B: Methanol / 0.1 % Trifluoressigsäure
Temperatur: 60°C
Gradient:

| Zeit in min | %A | %B | Flussrate in mL/min |
|---|---|---|---|
| 0.0 | 95 | 5 | 4.0 |
| 0.15 | 95 | 5 | 4.0 |
| 1.7 | 0 | 100 | 4.0 |
| 2.25 | 0 | 100 | 4.0 |

### Methode 4:

Säule: Agilent, StableBond, C18, 3 x 30 mm; 1.8 µm
Fließmittel A: Wasser / 0.1% Trifluoressigsäure
Fließmittel B: Acetonitril
Temperatur: 60°C
Gradient:

| Zeit in min | %A | %B | Flussrate in mL/min |
|---|---|---|---|
| 0.0 | 95 | 5 | 2.2 |
| 0.05 | 95 | 5 | 2.2 |
| 1.40 | 0 | 100 | 2.2 |
| 1.80 | 0 | 100 | 2.2 |

### Herstellung der Endverbindungen

### Beispiel 1

### (S)-6-Oxo-1,6-dihydro-pyridazin-4-carbonsäure- {3-[2-fluor-4-(4-methoxy-2-trifluormethyl-phenylamino)-benzylcarbamoyl]-tetrahydrofuran-3-yl}-amid

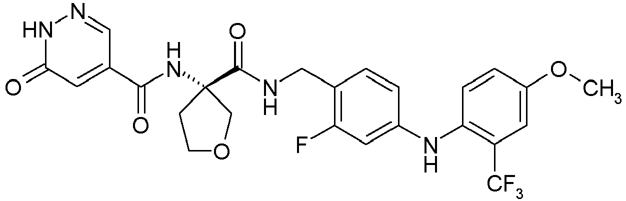

### 1 a) 2-Fluor-4-(4-methoxy-2-trifluormethyl-phenylamino)-benzonitril

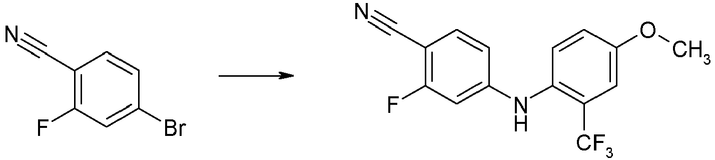

Zu einer Lösung von 4-Brom-2-fluor-benzonitril (10.46 mMol) in 50 mL Toluol wurden unter Stickstoff 2-Amino-5-methoxy-benzotrifluorid (10.46 mMol), K₃PO₄ (15.7 mMol), Xphos (1.05 mMol) und Pd₄(dba)₃ (0.314 mMol) gegeben und das Gemisch 20 Stunden bei 110°C Badtemperatur gerührt. Danach wurde das Gemisch über ein Glasfaserfilter filtriert, dann das Filtrat mit 150 mL Wasser ausgeschüttelt. Die organische Phase wurde über Natriumsulfat getrocknet und eingedampft. Man erhielt so das Produkt in einer Ausbeute von 87% der Theorie.
C₁₅H₁₀F₄N₂O (310.2)
Massenspektrum (ESI): [M+H]+ = 311
[M-H]- = 309
Dünnschichtchromatogramm (Kieselgel; Petroether / Essigsäureethylester 7 : 3): R_{f} = 0.48

### 1b) 2-Fluor-4-(4-methoxy-2-trifluormethyl-phenylamino)-benzylamin

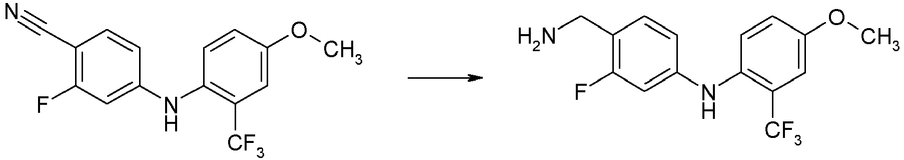

2-Fluor-4-(4-methoxy-2-trifluormethyl-phenylamino)-benzonitril (2.85 g, 9.19 mMol) wurde in 40 mL gesättigter methanolischer Ammoniaklösung nach Zugabe von 300 mg Raney-Nickel bei Raumtemperatur hydriert. Nach Abfiltrieren des Katalysators und Eindampfen erhielt man das Produkt in einer Ausbeute von 99% der Theorie.
C₁₅H₁₄F₄N₂O (314.3)
Dünnschichtchromatogramm (Kieselgel; Dichlormethan / Ethanol 19 : 1): R_{f} = 0.21

### 1c) (S)-3-[(6-Oxo-1,6-dihydro-pyridazin-4-carbonyl)-amino]-tetrahydrofuran-3-carbon-säure-n-butylester

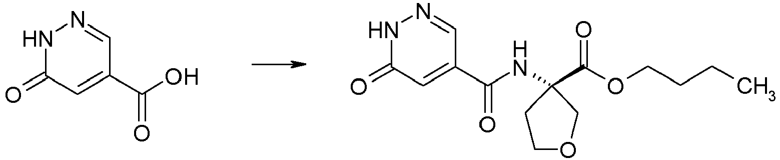

Eine Lösung von 6-Oxo-1,6-dihydro-pyridazin-4-carbonsäure (10.5 g, 74.9 mMol), TBTU (25.3 g, 78.7 mMol), Triethylamin (20.9 mL) und 40 mL DMF in 200 mL THF wurde 30 Minuten bei Raumtemperatur gerührt. Dann wurde (S)-3-Amino-tetrahydrofuran-3-carbon-säure-n-butylester (14.0 g, 74.9 mMol) hinzugegeben und weiter über Nacht gerührt. Zur Aufarbeitung wurde das Gemisch bis zur Trockne im Vakuum eingedampft und der Rückstand mit 200 mL Essigsäureethylester verrührt. Diese Lösung wurde zweimal mit 5%iger Natriumhydrogencarbonat-Lösung gewaschen, dann getrocknet und eingedampft. Man erhielt so das Produkt in einer Ausbeute von 90% der Theorie.
C₁₄H₁₉N₃O₅ (309.3)
Dünnschicht-Chromatogramm (Kieselgel; Dichlormethan/Ethanol 19 : 1): R_{f}= 0.16

### 1d) (S)-3-[(6-Oxo-1,6-dihydro-pyridazin-4-carbonyl)-amino]-tetrahydro-furan-3-carbon-säure

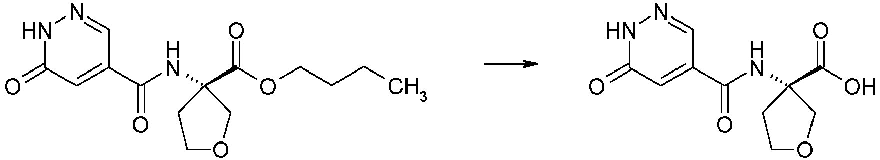

(*S*)-3-[(6-Oxo-1,6-dihydro-pyridazin-4-carbonyl)-amino]-tetrahydrofuran-3-carbonsäure-*n*-butylester (21.0 g, 67.9 mMol) wurde in 200 mL 1N Natronlauge 1 Stunde heftig gerührt. Das Gemisch wurde dann zweimal mit je 100 mL Diethylether extrahiert, die alkalische wässrige Phase dann mit 50 mL 4N Salzsäure versetzt. Das Gemisch wurde dann bis zur Trockne eingedampft und der Rückstand mit 150 mL Ethanol verrührt. Ungelöste Bestandteile wurden dann abfiltriert und das Filtrat eingedampft. Man erhielt so das Produkt in einer Ausbeute von 71% der Theorie. Das so erhaltene Produkt wurde ohne Reinigung weiter verarbeitet.
C₁₀H₁₁N₃O₅ (253.2)
¹H-NMR (d₆-DMSO): δ = 2.32 (m, 2H); 3.84 (t, 2H); 3.95 (d, 1 H); 4.12 (d, 1 H); 7.28 (s, 1 H); 8.10 (s, 1 H); 9.21 (breites S; 1 H).

### 1e) (S)-6-Oxo-1,6-dihydro-pyridazin-4-carbonsäure- {3-[2-fluor-4-(4-methoxy-2-tri-fluormethyl-phenylamino)-benzylcarbamoyl]-tetrahydrofuran-3-yl}-amid

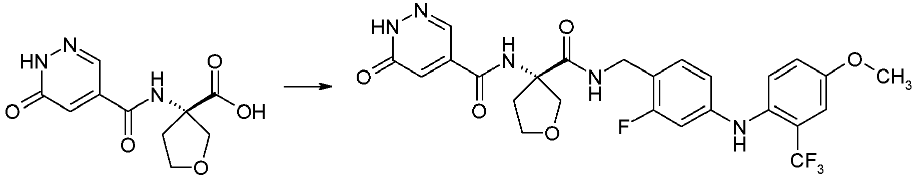

(*S*)-3-[(6-Oxo-1,6-dihydro-pyridazin-4-carbonyl)-amino]-tetrahydrofuran-3-carbonsäure (0.55 mMol) wurde in einem Gemisch aus 30 mL Tetrahydrofuran, 4 mL DMF und 0.15 mL Triethylamin gelöst, dann wurde TBTU (0.19 g, 0.58 mMol) zugegeben und 30 Minuten bei Raumtemperatur gerührt. Danach wurde 2-Fluor-4-(4-methoxy-2-trifluormethyl-phenylamino)-benzylamin (0.17 g, 0.55 mMol, aus 1 b) hinzugefügt und weitere zwei Stunden gerührt. Da noch nicht-umgesetztes Amin enthalten war, wurden noch 10 mg der Säure und 20 mg TBTU hinzugegeben und über Nacht weitergerührt. Danach wurden die Lösungsmittel abgedampft und der Rückstand über Kieselgel chromatographiert (Laufmittel: Dichlormethan / Methanol / Ammoniak: 95 / 5 / 0.5). Man erhielt so das Produkt in einer Ausbeute von 19% der Theorie.
C₂₅H₂₃F₄N₅O₅ (549.5)
Massenspektrum (ESI): [M+H]+ = 550
[M-H]- = 548
Dünnschichtchromatogramm (Kieselgel; Dichlormethan / Methanol / Ammoniak: 9 / 1 / 0.1): R_{f} = 0.46

### Beispiel 2

### (S)-5-Amino-N-{3-[2-fluor-4-(4-methoxy-2-trifluormethyl-phenylamino)-benzylcarbamoyl]-tetrahydro-furan-3-yl}-nicotinamid

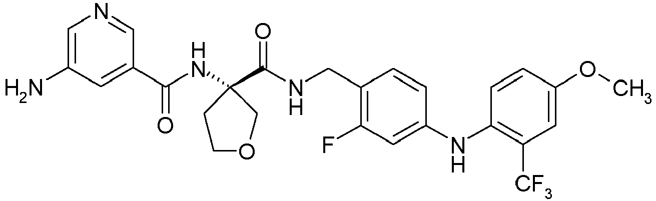

### 2a) (S)-3-[(5-Amino-pyridin-3-carbonyl)-amino]-tetrahydrofuran-3-carbonsäuren-butylester

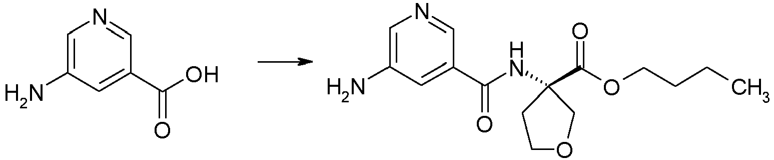

Analog Beispiel 1c) wurde 5-Aminopyridin-3-carbonsäure (72.4 mMol) mit (*S*)-3-Amino-tetrahydrofuran-3-carbonsäure-*n*-butylester (72.4 mMol) umgesetzt. Man erhielt das Produkt in einer Ausbeute von 96% der Theorie.
C₁₅H₂₁N₃O₄ (307.3)
Massenspektrum (ESI): [M+H]+ = 308
[M-H]- = 306
Dünnschichtchromatogramm (Kieselgel; Essigsäureethylester / Ethanol 9 : 1): R_{f} = 0.58

### 2b) (S)-3-[(5-Amino-pyridin-3-carbonyl)-amino]-tetrahydrofuran-3-carbonsäure

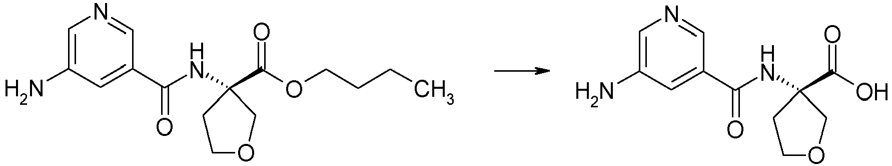

Analog Beispiel 1d) wurde (*S*)-3-[(5-Amino-pyridin-3-carbonyl)-amino]-tetrahydrofuran-3-carbonsäure-*n*-butylester (69.9 mMol) mit Natronlauge verseift. Man erhielt das Produkt in einer Ausbeute von 86% der Theorie.
C₁₁ H₁₃N₃O₄ (251.2)
Massenspektrum (ESI): [M+H]+ = 252
¹H-NMR (d₆-DMSO): δ = 2.33 (m, 2H); 3.82 (m, 2H); 5.48 (breites s, 2H); 7.27 (s, 1H); 8.03 (s, 1 H); 8.18 (s, 1 H); 8.85 (s, 1 H); 12.60 (breites s, 1 H) ppm.

### 2c) (S)-5-Amino-N-{3-[2-fluor-4-(4-methoxy-2-trifluormethyl-phenylamino)-benzyl-carbamoyl]-tetrahydro-furan-3-yl}-nicotinamid

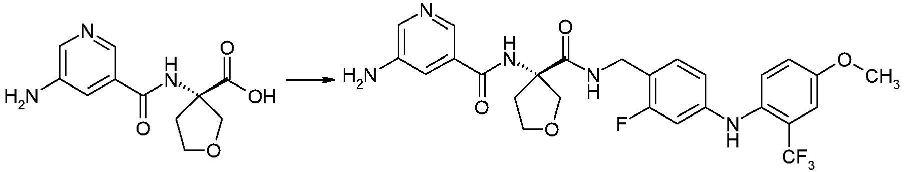

Analog Beispiel 1e) wurde (*S*)-3-[(5-Amino-pyridin-3-carbonyl)-amino]-tetrahydrofuran-3-carbonsäure (0.55 mMol) mit 2-Fluor-4-(4-methoxy-2-trifluormethyl-phenylamino)-benzylamin (0.55 mMol, aus 1 b) umgesetzt. Man erhielt das Produkt in einer Ausbeute von 27% der Theorie.
C₂₆H₂₅F₄N₅O₄ (547.5)
Massenspektrum (ESI): [M+H]+ = 548
[M-H]- = 546
HPLC: Rₜ= 2.44 min (Methode 1)

### Beispiel 3

### (S)-5-Amino-N-(3-{[3-fluor-5-(4-methoxy-2-trifluormethyl-phenylamino)-pyridin-2-ylmethyl]-carbamoyl}-tetrahydrofuran-3-yl)-nicotinamid

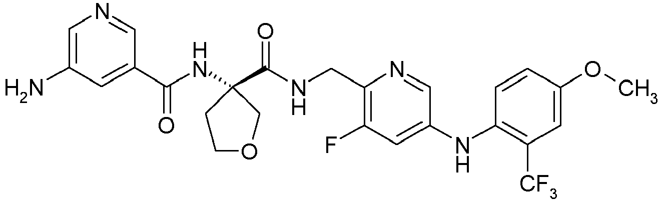

### 3a) (5-Brom-3-fluor-pyridin-2-ylmethyl)-carbaminsäure-tert-butylester

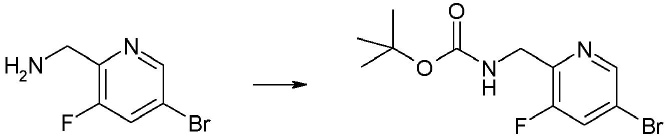

Eine Lösung von 2-Aminomethyl-3-fluor-5-brompyridin (185 mg, 0.77mMol) in 8 mL Dichlormethan wurde unter Eisbadkühlung mit 0.32 mL Triethylamin und Di-*tert*-butyl-dicarbonat (167.2 mg, 0.77 mMol) versetzt und anschließend über Nacht bei Raumtemperatur gerührt. Nach Standard-Aufarbeitung des Reaktionsansatzes erhielt man das Produkt in einer Ausbeute von 72% der Theorie.
C₁₁H₁₄BrFN₂O₂ (305.14)
MS (ESI): [M+H]+ = 305/7
HPLC: Rₜ= 2.31 min (Methode 1)

### 3b) [3-Fluor-5-(4-methoxy-2-trifluormethyl-phenylamino)-pyridin-2-ylmethyl]-carbaminsäure-tert-butylester

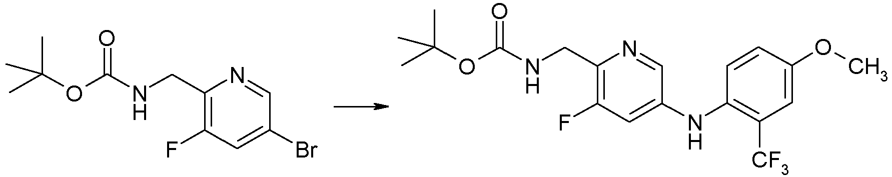

Analog Beispiel 1a) wurde (5-Brom-3-fluor-pyridin-2-ylmethyl)-carbaminsäure-*tert-*butylester (0.55 mMol) mit 4-Methoxy-2-trifluormethyl-anilin umgesetzt. Das Rohprodukt wurde chromatographisch gereinigt (Säule: Varian Pursuit XRS C18; 10 µM; 41.4 x 250 mm. Gradient: Acetonitril / Wasser /CF₃COOH: 10 / 90 / 0.1 →100/0/0.1). Man erhielt so das Produkt in einer Ausbeute von 26% der Theorie.
C₁₉H₂₁ F₄N₃O₃ (415.4)
MS (ESI): [M+H]+ = 416
HPLC: Rₜ= 2.77 min (Methode 1)

### 3c) (6-Aminomethyl-5-fluor-pyridin-3-yl)-(4-methoxy-2-trifluormethyl-phenyl)-amin

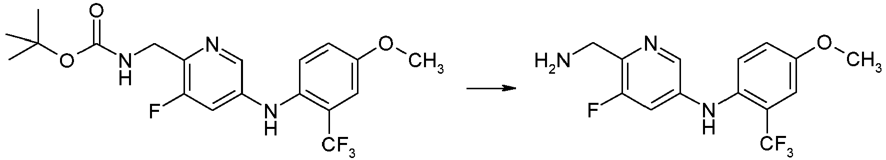

[3-Fluor-5-(4-methoxy-2-trifluormethyl-phenylamino)-pyridin-2-ylmethyl]-carbaminsäure-*tert*-butylester (0.13 mMol) wurde zwei Stunden bei 60°C in einem Gemisch aus 2 mL halbkonzentrierter Salzsäure und 3 mL Dioxan gerührt. Danach wurde bis zur Trockne eingedampft, dann der Rückstand mit 3 mL Toluol verrührt und erneut eingedampft. Das so erhaltene Rohprodukt (89% der Theorie) wurde ohne Reinigung weiter eingesetzt.
C₁₄H₁₃F₄N₃O (315.3)
MS (ESI): [M+H]+ = 316

### 3d) (S)-5-Amino-N-(3-{[3-fluor-5-(4-methoxy-2-trifluormethyl-phenylamino)-pyridin-2-yl-methyl]-carbamoyl}-tetrahydro-furan-3-yl)-nicotinamid

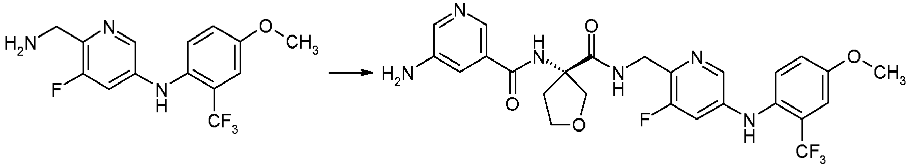

Analog Beispiel 1e) wurde (*S*)-3-[(5-Amino-pyridin-3-carbonyl)-amino]-tetrahydrofuran-3-carbonsäure (aus 2b) mit (6-Aminomethyl-5-fluor-pyridin-3-yl)-(4-methoxy-2-trifluormethyl-phenyl)-amin umgesetzt. Man erhielt das Produkt in einer Ausbeute von 34% der Theorie.
C₂₅H₂₄F₄N₆O₄ (548.5)
MS (ESI): [M+H]+ = 549
HPLC: Rₜ= 2.55 min (Methode 1)

### Beispiel 4

### (S)-5-Amino-N-{3-[4-(4-methoxy-2-trifluormethyl-phenylamino)-benzylcarbamoyl]-tetrahydrofuran-3-yl}-nicotinamid

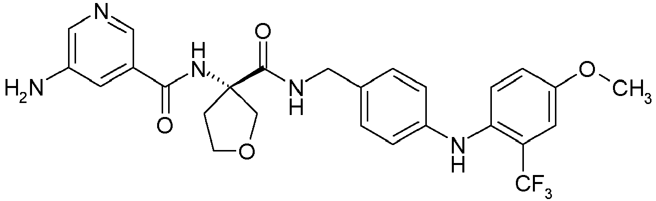

### 4a) 4-(4-Methoxy-2-trifluormethyl-phenylamino)-benzonitril

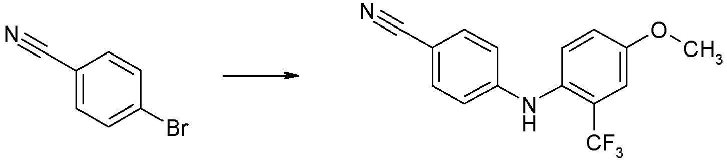

Analog zu Beispiel 1a) wurde 4-Brombenzonitril mit 2-Trifluormethyl-4-methoxy-anilin umgesetzt. Nach chromatographischer Reinigung über Kieselgel (Petrolether mit 10 bis 30% Essigsäureethylester) erhielt man das 4-(4-Methoxy-2-trifluormethyl-phenylamino)-benzonitril in einer Ausbeute von 60.5% der Theorie.
C₁₅H₁₁F₃N₂O (292.3)
Massenspektrum (ESI): [M+H]⁺ = 293
[M-H]⁻ = 291

### 4b) (4-Aminomethyl-phenyl)-(4-methoxy-2-trifluormethyl-phenyl)-amin

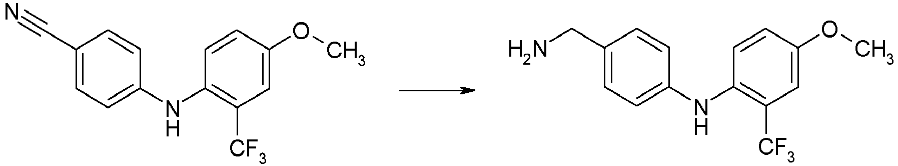

3.7 g (12.7 mMol) 4-(4-Methoxy-2-trifluormethyl-phenylamino)-benzonitril wurden in einer 7N-Lösung von Ammoniak in Methanol (100 mL) unter Zusatz von Raney-Nickel bei Raumtemperatur hydriert. Nach Abfiltrieren des Katalysators und Abdestillieren des Lösungsmittels wurde das so erhaltene Rohprodukt ohne Reinigung weiter umgesetzt. C₁₅H₁₅F₃N₂O (296.3)

### 4c) (S)-5-Amino-N-{3-[4-(4-methoxy-2-trifluormethyl-phenylamino)-benzylcarbamoyl]-tetrahydrofuran-3-yl}-nicotinamid

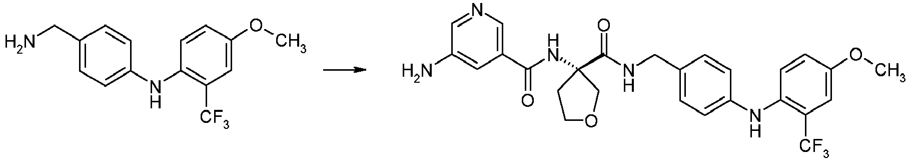

Analog zu Beispiel 1 e) wurde (S)-3-[(5-Amino-pyridin-3-carbonyl)-amino]-tetrahydrofuran-3-carbonsäure (1.00 mMol) mit (4-Aminomethyl-phenyl)-(4-methoxy-2-trifluormethylphenyl)-amin (1.00 mMol, aus Beispiel 4b)) umgesetzt. Man erhielt das Produkt in einer Ausbeute von 32% der Theorie.
C₂₆H₂₆F₃N₅O₄ (529.5)
Massenspektrum (ESI): [M+H]+ = 530
[M-H]- = 528
HPLC: Rₜ= 0.842 min (Methode 4)
Dünnschichtchromatogramm (Kieselgel; Dichlormethan / Ethanol 9 : 1 + 1% NH₄OH): R_{f} = 0.25

### Beispiel 5

### (S)-5-Amino-N-(3-{[5-(4-methoxy-2-trifluormethyl-phenylamino)-pyridin-2-ylmethyl]-carbamoyl}-tetrahydrofuran-3-yl)-nicotinamid

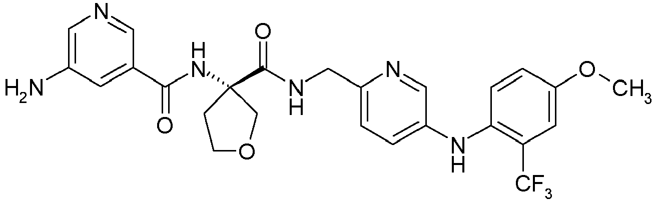

### 5a) 5-(4-Methoxy-2-trifluormethyl-phenylamino)-pyridin-2-carbonitril

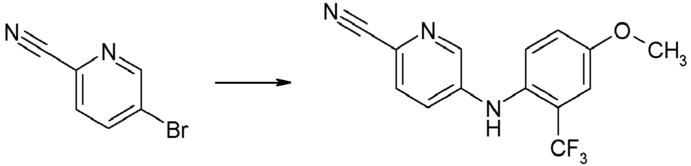

Analog zu Beispiel 1a) wurde 5-Brom-pyridin-2-carbonitril mit 2-Trifluormethyl-4-methoxy-anilin umgesetzt. Nach chromatographischer Reinigung über Kieselgel (Petrolether mit 10 bis 30% Essigsäureethylester) erhielt man das 4-(4-Methoxy-2-trifluormethyl-phenyl-amino)-benzonitril in einer Ausbeute von 97.4% der Theorie.
C₁₄H₁₀F₃N₃O (293.2)
Massenspektrum (ESI): [M+H]⁺ = 294
[M-H]⁻ = 292
¹H-NMR (d₆-DMSO): δ = 3.87 (s, 3H); 6.88 (dd, 1H); 7.31 (m, 2H); 7.47 (d, 1H); (7.67 (d, 1 H); 8.11 (s, 1 H); 8.62 (s, 1 H) ppm.

### 5b) (6-Aminomethyl-pyridin-3-yl)-(4-methoxy-2-trifluormethyl-phenyl)-amin

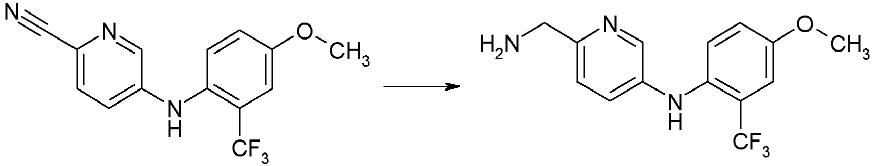

Analog zu Beispiel 4b wurde 5-(4-Methoxy-2-trifluormethyl-phenylamino)-pyridin-2-carbo-nitril (1.71 mMol) in einer 7N-Lösung von Ammoniak in Methanol (30 mL) unter Zusatz von Raney-Nickel bei Raumtemperatur hydriert. Nach Abfiltrieren des Katalysators und Abdestillieren des Lösungsmittels wurde das so erhaltene Rohprodukt ohne Reinigung weiter umgesetzt.
C₁₄H₁₄F₃N₃O (297.3)
Dünnschichtchromatogramm (Kieselgel; Dichlormethan / Ethanol 9 : 1): R_{f}= 0.11

### 5c) (S)-3-[(5-tert-Butoxycarbonylamino-pyridin-3-carbonyl)-amino]-tetrahydrofuran-3-carbonsäure-n-butylester

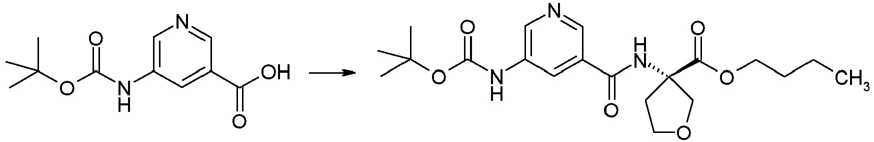

Eine Suspension von 5-*tert*-Butoxycarbonylamino-pyridin-3-carbosäure (2.5 mMol) in 20 mL Tetrahydrofuran wurde mit *N,N*-Carbodiimidazol (2.75 mMol) versetzt und 15 Minuten bei Raumtemperatur gerührt. Dann wurde eine Lösung von (*S*)-3-Amino-tetrahydrofuran-3-carbonsäure-*n*-butylester (2.5 mMol) in 4 mL Tetrahydrofuran hinzugefügt und das Reaktionsgemisch über Nacht weiter gerührt. Danach wurde das Lösungsmittel abdestilliert und das so erhaltene Rohprodukt chromatographisch gereinigt (Kieselgel; Dichlormethan mit 1-25% Ethanol).
Ausbeute: 31 % der Theorie
C₂₀H₂₉N₃O₆ (407.5)
Massenspektrum (ESI): [M+H]⁺ = 408
[M-H]⁻ = 406

### 5d) (S)-3-[(5-tert-Butoxycarbonylamino-pyridin-3-carbonyl)-amino]-tetrahydrofuran-3-carbonsäure

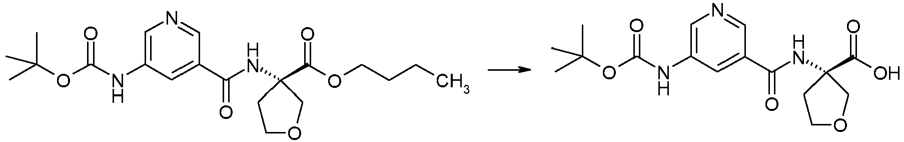

Eine Lösung von (*S*)-3-[(5-*tert*-Butoxycarbonylamino-pyridin-3-carbonyl)-amino]-tetra-hydrofuran-3-carbonsäure-n-butylester (29.7 mMol) in 150 mL Methanol wurde mit 60 mL 1 N Natronlauge versetzt und drei Stunden bei Raumtemperatur gerührt. Danach wurde das Methanol abdestilliert, die Lösung dann mit 50 mL tert-Butylmethylether gewaschen und anschließend mit 4N Salzsäure auf pH 3 eingestellt. Das daraufhin ausgefallene Produkt wurde abfiltriert und ohne Reinigung weiter umgesetzt.
Ausbeute: 94% der Theorie
C₁₆H₂₁N₃O₆ (351.4)
Massenspektrum (ESI): [M+H]⁺ = 352
[M-H]⁻ = 350

### 5e) (S)-[5-(3-{[5-(4-Methoxy-2-trifluormethyl-phenylamino)-pyridin-2-ylmethyl]-carbamoyl}-tetrahydrofuran-3-ylcarbamoyl)-pyridin-3-yl]-carbaminsäure-tert-butyl-ester

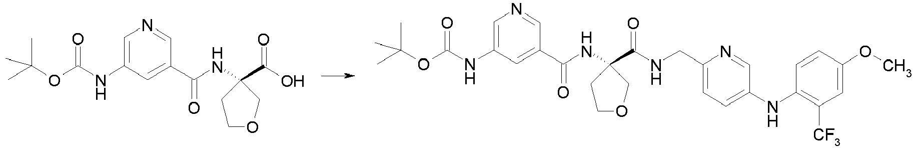

Analog zu Beispiel 1e) wurde (*S*)-3-[(5-*tert*-Butoxycarbonylamino-pyridin-3-carbonyl)-amino]-tetrahydrofuran-3-carbonsäure (Produkt aus Beispiel 5d, 1.7 mMol) mit (6-Amino-methyl-pyridin-3-yl)-(4-methoxy-2-trifluormethyl-phenyl)-amin (Produkt aus Beispiel 5b, 1.7 mMol) umgesetzt. Das Produkt wurde chromatographisch gereinigt (Kieselgel, Dichlormethan mit 0-10% Methanol).
Ausbeute: 69% der Theorie
C₃₀H₃₃F₃N₆O₆ (630.6)
Dünnschichtchromatogramm (Kieselgel; Dichlormethan / Ethanol 9 : 1): R_{f} = 0.46

### 5f) (S)-5-Amino-N-(3-{[5-(4-methoxy-2-trifluormethyl-phenylamino)-pyridin-2-ylmethyl]-carbamoyl}-tetrahydrofuran-3-yl)-nicotinamid

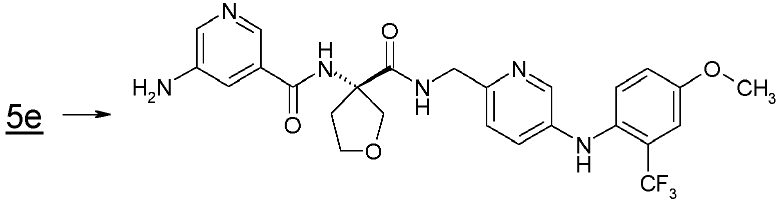

(*S*)-[5-(3-{[5-(4-Methoxy-2-trifluormethyl-phenylamino)-pyridin-2-ylmethyl]-carbamoyl}-tetrahydrofuran-3-ylcarbamoyl)-pyridin-3-yl]-carbaminsäure-tert-butylester (Produkt aus Beispiel 5e, 1.2 mMol) wurde mit einer 4N HCl-Lösung in Dioxan (20 mL) zwei Stunden bei Raumtemperatur gerührt. Anschließend wurde bis zur Trockne eingedampft, der Rückstand mit ca. 20 mL Diethylether verrieben und abgesaugt.
Ausbeute: 82% der Theorie
C₂₅H₂₅F₃N₆O₄ (530.5)
Massenspektrum (ESI): [M+H]⁺ = 531
[M-H]⁻ = 529
HPLC: Rₜ= 1.014 min (Methode 3)

### Beispiel 6

### (S)-5-Amino-N-(3-{[3-chlor-5-(4-methoxy-2-trifluormethyl-phenylamino)-pyridin-2-yl-methyl]-carbamoyl}-tetrahydrofuran-3-yl)-nicotinamid

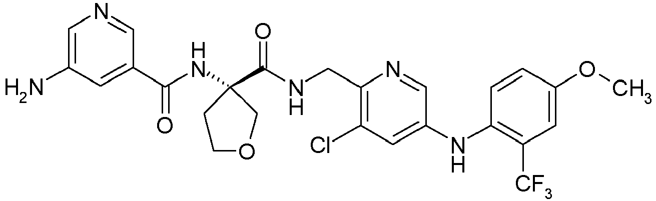

### 6a) 3-Chlor-5-(4-methoxy-2-trifluormethyl-phenylamino)-pyridin-2-carbonitril

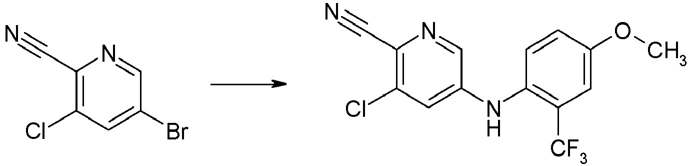

5-Brom-3-chlor-pyridin-2-carbonitril (2.3 mMol) wurde analog zu Beispiel 1 a) mit 4-Methoxy-2-trifluoranilin (2.3 mMol) umgesetzt. Nach chromatographischer Reinigung über Kieselgel (Petrolether mit 15 bis 30% Essigsäureethylester) erhielt man das Produkt in einer Ausbeute von 27% der Theorie.
C₁₄H₉CIF₃N₃O (327.7)
Massenspektrum (ESI): [M+H]⁺ = 328
[M-H]⁻ = 326
HPLC: Rₜ= 1.558 min (Methode 2)

### 6b) (6-Aminomethyl-5-chlor-pyridin-3-yl)-(4-methoxy-2-trifluormethyl-phenyl)-amin

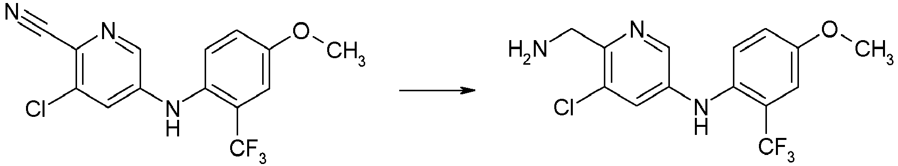

Eine Lösung von 3-Chlor-5-(4-methoxy-2-trifluormethyl-phenylamino)-pyridin-2-carbonitril (0.64 mMol) in 10 mL Ethanol und 0.1 mL Salzsäure (37%) wurde mit 30 mg Pd/Kohle (10%) versetzt und unter Rühren bei Raumtemperatur hydriert. Anschließend wurde der Katalysator abfiltriert und die Lösung eingedampft. Das so erhaltene Produkt wurde ohne Reinigung weiter umgesetzt.
Ausbeute: 99% der Theorie
C₁₄H₁₃ClF₃N₃O (331.7)
HPLC: Rₜ= 1.167 min (Methode 2)

### 6c) (S)-5-Amino-N-(3-{[3-chlor-5-(4-methoxy-2-trifluormethyl-phenylamino)-pyridin-2-ylmethyl]-carbamoyl}-tetrahydrofuran-3-yl)-nicotinamid

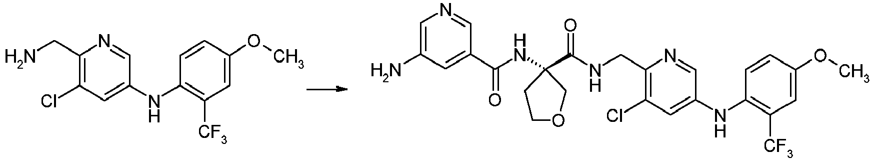

Analog zu Beispiel 1 e) wurde (6-Aminomethyl-5-chlor-pyridin-3-yl)-(4-methoxy-2-trifluormethyl-phenyl)-amin (1.3 mMol) mit (S)-3-[(5-Amino-pyridin-3-carbonyl)-amino]-tetrahydrofuran-3-carbonsäure (Produkt aus Beispiel 2b, 1.3 mMol) umgesetzt. Nach chromatographischer Reinigung (Kieselgel, Dichlormethan/Methanol 9:1 mit 3-8% Ammoniak) wurde das Produkt in einer Ausbeute von 13% der Theorie erhalten.
C₂₅H₂₄C1F₃N₆O₄ (564.9)
¹H-NMR (d₆-DMSO): δ = 2.27-2.46 (m, 2H); 3.78 - 3.88 (m, 2H); 3.85 (s, 3H); 3.94 (m, 1 H); 4.23 (m, 1 H); 4.24 - 4.38 (m, 2H); 5.49 (m 2H); 6.97 (d, 1H); 7.27-7.31 (m, 3H); 7.35-7.41 (m, 1H); 7.85 (d, 2H); 7.95 (m, 1H); 8.04 (d, 1H); 8.23 (s, 1H); 8.80 (s, 1H) ppm.

Die nachfolgenden Beispiele beschreiben pharmazeutische Darreichungsformen, die als Wirkstoff eine beliebige Verbindung der allgemeinen Formel I enthalten, ohne jedoch den Umfang der vorliegenden Erfindung darauf einzuschränken:

### Beispiel I

### Trockenampulle mit 75 mg Wirkstoff pro 10 ml

### Zusammensetzung:

| | |
|---|---|
| Wirkstoff | 75.0 mg |
| Mannitol | 500 mg |
| Wasser für Injektionszwecke ad | 10.0 ml |

### Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

### Beispiel II

### Tablette mit 50 mg Wirkstoff

### Zusammensetzung:

| | |
|---|---|
| (1) Wirkstoff | 50.0 mg |
| (2) Milchzucker | 98.0 mg |
| (3) Maisstärke | 50.0 mg |
| (4) Polyvinylpyrrolidon | 15.0 mg |
| (5) Magnesiumstearat | 2.0 mg |
| | 215.0 mg |

### Herstellung:

(1), (2) und (3) werden gemischt und mit einer wässrigen Lösung von (4) granuliert. Dem getrockneten Granulat wird (5) zugemischt. Aus dieser Mischung werden Tabletten gepresst, biplan mit beidseitiger Facette und einseitiger Teilkerbe.
Durchmesser der Tabletten: 9 mm.

### Beispiel III

### Tablette mit 350 mg Wirkstoff

### Zusammensetzung:

| | |
|---|---|
| (1) Wirkstoff | 350.0 mg |
| (2) Milchzucker | 136.0 mg |
| (3) Maisstärke | 80.0 mg |
| (4) Polyvinylpyrrolidon | 30.0 mg |
| (5) Magnesiumstearat | 4.0 mg |
| | 600.0 mg |

### Herstellung:

(1), (2) und (3) werden gemischt und mit einer wässrigen Lösung von (4) granuliert. Dem getrockneten Granulat wird (5) zugemischt. Aus dieser Mischung werden Tabletten gepresst, biplan mit beidseitiger Facette und einseitiger Teilkerbe.
Durchmesser der Tabletten: 12 mm.

### Beispiel IV

### Kapseln mit 50 mg Wirkstoff

### Zusammensetzung:

| | |
|---|---|
| (1) Wirkstoff | 50.0 mg |
| (2) Maisstärke getrocknet | 58.0 mg |
| (3) Milchzucker pulverisiert | 50.0 mg |
| (4) Magnesiumstearat | 2.0 mg |
| | 160.0 mg |

### Herstellung:

(1) wird mit (3) verrieben. Diese Verreibung wird der Mischung aus (2) und (4) unter intensiver Mischung zugegeben.
Diese Pulvermischung wird auf einer Kapselabfüllmaschine in Hartgelatine-Steckkapseln Größe 3 abgefüllt.

### Beispiel V

### Kapseln mit 350 mg Wirkstoff

### Zusammensetzung:

| | |
|---|---|
| (1) Wirkstoff | 350.0 mg |
| (2) Maisstärke getrocknet | 46.0 mg |
| (3) Milchzucker pulverisiert | 30.0 mg |
| (4) Magnesiumstearat | 4.0 mg |
| | 430.0 mg |

### Herstellung:

(1) wird mit (3) verrieben. Diese Verreibung wird der Mischung aus (2) und (4) unter intensiver Mischung zugegeben.
Diese Pulvermischung wird auf einer Kapselabfüllmaschine in Hartgelatine-Steckkapseln Gr6Be 0 abgefüllt.

### Beispiel VI

### Suppositorien mit 100 mg Wirkstoff

| 1 Zäpfchen enthält: | |
|---|---|
| Wirkstoff | 100.0 mg |
| Polyethylenglykol (M.G. 1500) | 600.0 mg |
| Polyethylenglykol (M.G. 6000) | 460.0 mg |
| Polyethylensorbitanmonostearat | 840.0 mg |
| | 2000.0 mg |

## Patentansprüche

1. Verbindungen der allgemeinen Formel **I** 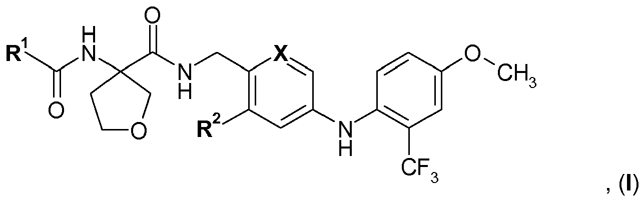 in der
**R¹** eine Gruppe ausgewählt aus 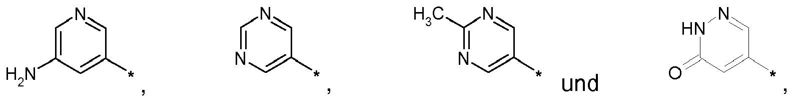
**R²** H, Cl oder F und
**X** CH oder N bedeuten,
deren Enantiomere, deren Diastereomere, deren Gemische und deren Salze, insbesondere deren physiologisch verträgliche Salze mit organischen oder anorganischen Säuren oder Basen.

2. Verbindungen nach Anspruch 1, nämlich Verbindungen der allgemeinen Formel **la** 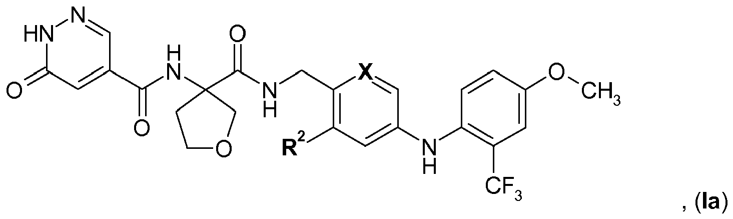 in der
**R²** H, Cl oder F und
**X** CH oder N bedeuten,
deren Enantiomere, deren Diastereomere, deren Gemische und deren Salze, insbesondere deren physiologisch verträgliche Salze mit organischen oder anorganischen Säuren oder Basen.

3. Verbindungen nach Anspruch 1, nämlich Verbindungen der allgemeinen Formel **Ib** 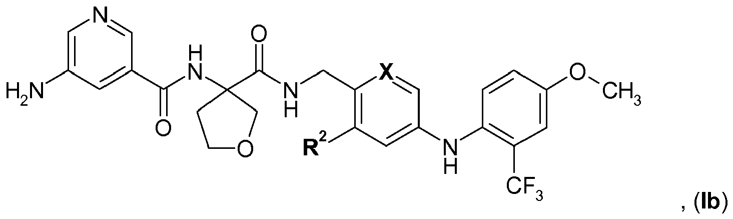 in der
**R²** H, Cl oder F und
**X** CH oder N bedeuten,
deren Enantiomere, deren Diastereomere, deren Gemische und deren Salze, insbesondere deren physiologisch verträgliche Salze mit organischen oder anorganischen Säuren oder Basen.

4. Verbindungen nach Anspruch 1, nämlich Verbindungen der allgemeinen Formel **Ic** 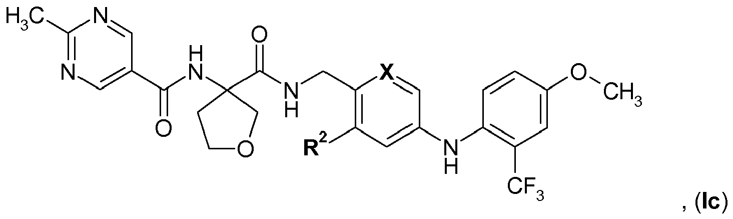 in der
**R²** H, Cl oder F und
**X** CH oder N bedeuten,
deren Enantiomere, deren Diastereomere, deren Gemische und deren Salze, insbesondere deren physiologisch verträgliche Salze mit organischen oder anorganischen Säuren oder Basen.

5. Verbindungen nach Anspruch 1, nämlich Verbindungen der allgemeinen Formel Id 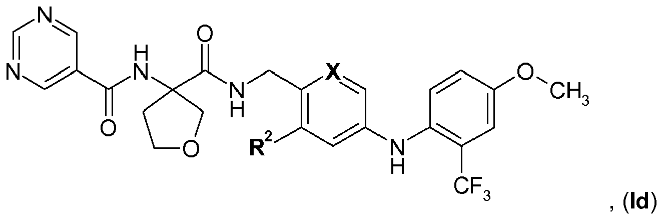 in der
**R²** H, Cl oder F und
**X** CH oder N bedeuten,
deren Enantiomere, deren Diastereomere, deren Gemische und deren Salze, insbesondere deren physiologisch verträgliche Salze mit organischen oder anorganischen Säuren oder Basen.

6. Verbindungen nach einem der vorherigen Ansprüche, nämlich 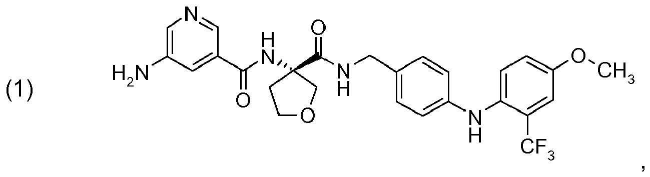 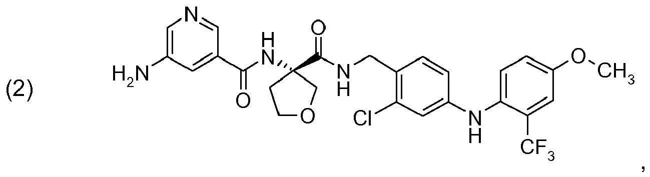 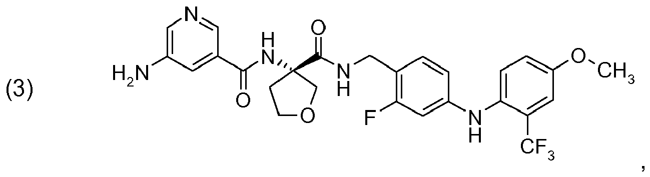 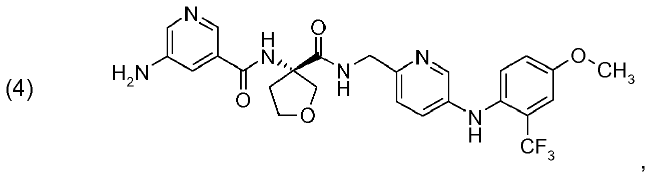 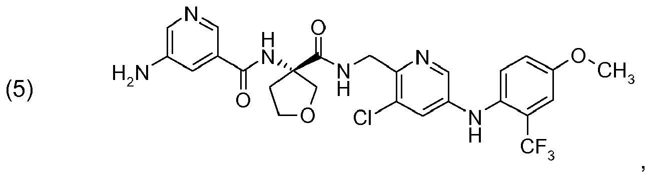 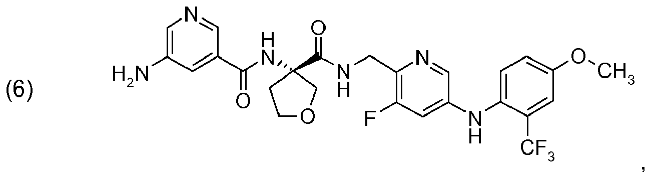 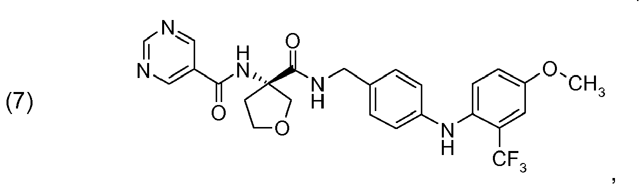 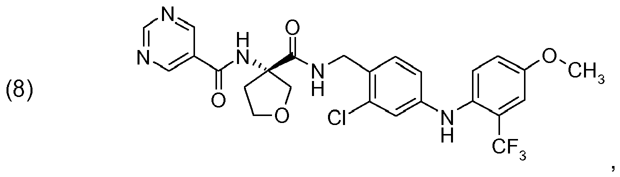 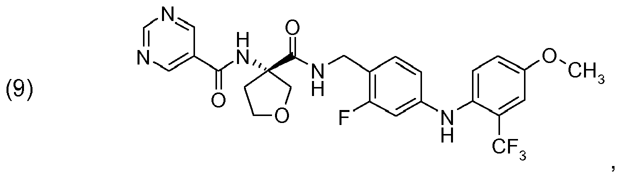 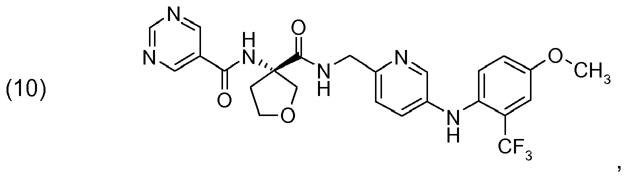 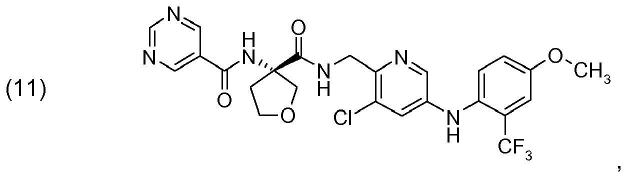 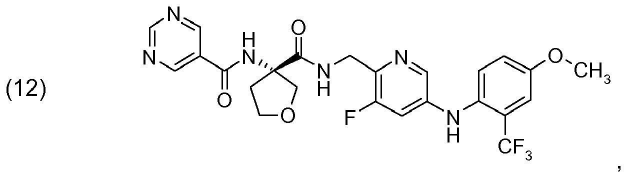 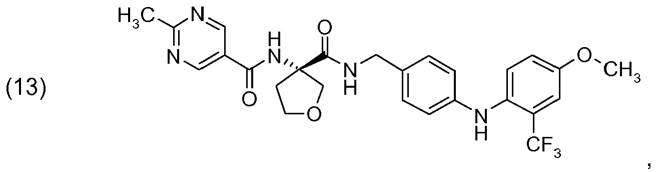 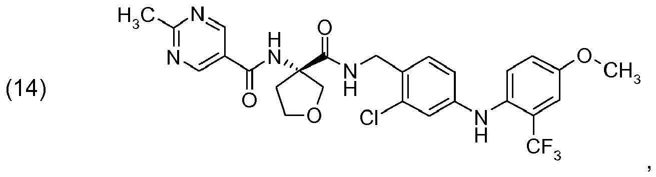 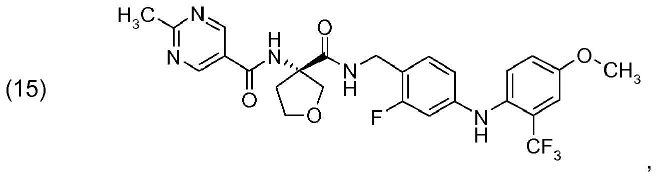 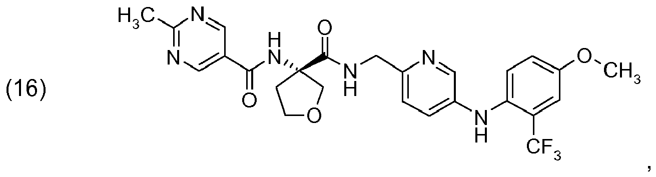 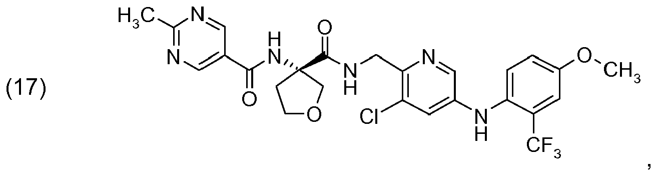 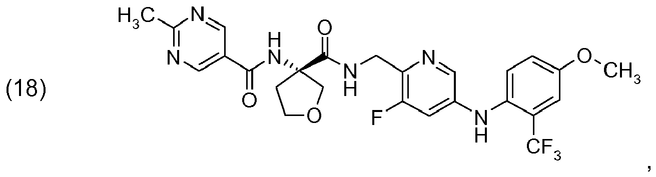 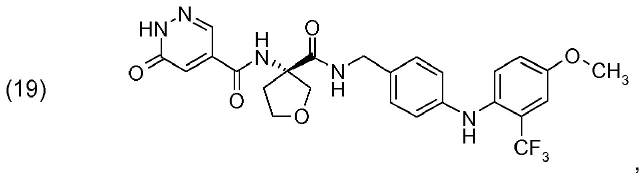 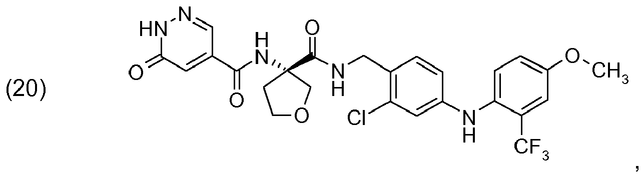 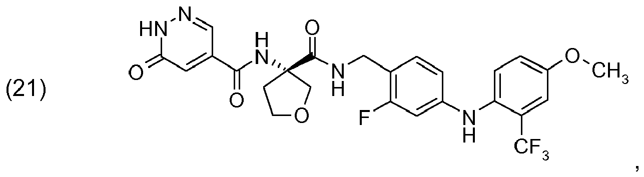 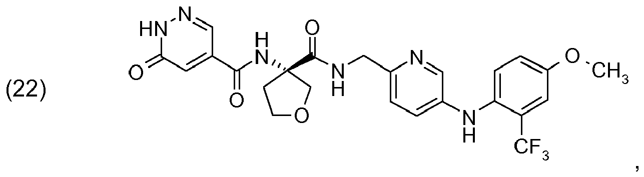 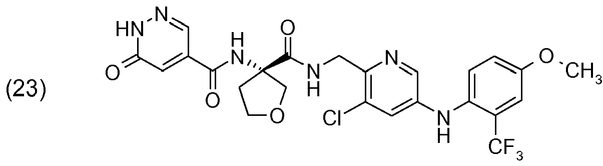 und 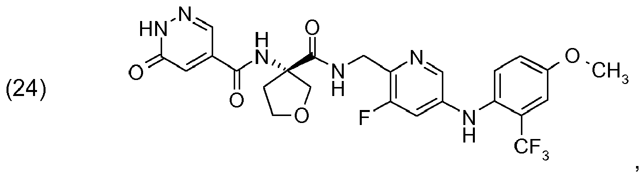 deren Enantiomere, deren Diastereomere, deren Gemische und deren Salze, insbesondere deren physiologisch verträgliche Salze mit organischen oder anorganischen Säuren oder Basen.

7. Physiologisch verträgliche Salze der Verbindungen nach einem der vorherigen Ansprüche mit anorganischen oder organischen Säuren oder Basen.

8. Pharmazeutische Zusammensetzungen enthaltend eine Verbindung nach einem der vorherigen Ansprüche, gegebenenfalls zusammen mit einem oder mehreren inerten Trägerstoffen und/oder Verdünnungsmitteln.

9. Arzneimittel enthaltend eine Verbindung nach einem der Ansprüche 1-7.

10. Eine Verbindung nach einem der Ansprüche 1-7 zur Anwendung in der akuten und prophylaktischen Behandlung von Osteoarthritis, akuten Schmerzen, Eingeweideschmerzen, neuropathischen Schmerzen, entzündlichen / Schmerzrezeptor-vermittelten Schmerzen, Tumorschmerzen, Kopfschmerz-Erkrankungen, diabetischer Neuropathie, chronischen Rückenschmerzen, Asthma, chronischer Bronchitis oder COPD.

## Claims

1. Compounds of general formula I 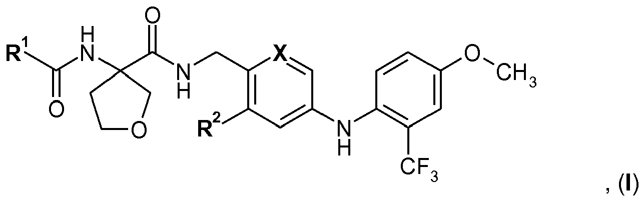 wherein
**R¹** denotes a group selected from 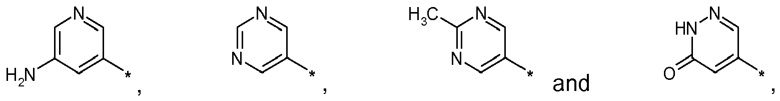
**R²** denotes H, Cl or F and
**X** denotes CH or N,
the enantiomers, the diastereomers, the mixtures and the salts thereof, particularly the physiologically acceptable salts thereof with organic or inorganic acids or bases.

2. Compounds according to claim 1, namely compounds of general formula la 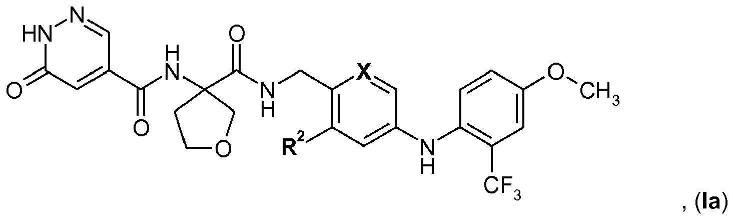 wherein
**R²** denotes H, Cl or F and
**X** denotes CH or N,
the enantiomers, the diastereomers, the mixtures and the salts thereof, particularly the physiologically acceptable salts thereof with organic or inorganic acids or bases.

3. Compounds according to claim 1, namely compounds of general formula **Ib** 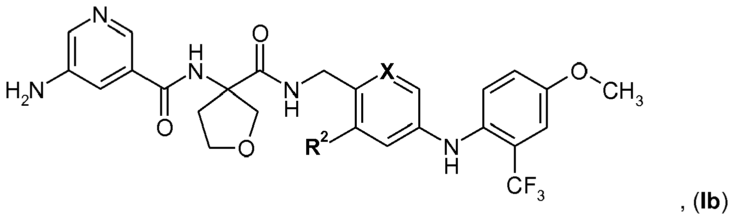 wherein
**R²** denotes H, Cl or F and
**X** denotes CH or N,
the enantiomers, the diastereomers, the mixtures and the salts thereof, particularly the physiologically acceptable salts thereof with organic or inorganic acids or bases.

4. Compounds according to claim 1, namely compounds of general formula **Ic** 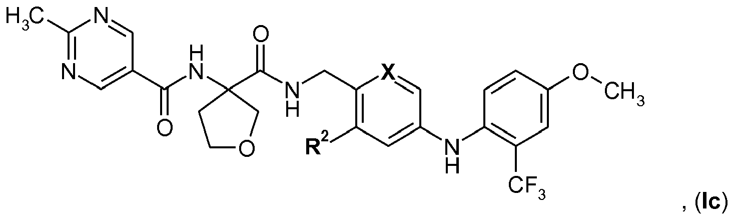 wherein
**R**² denotes H, Cl or F and
**X** denotes CH or N,
the enantiomers, the diastereomers, the mixtures and the salts thereof, particularly the physiologically acceptable salts thereof with organic or inorganic acids or bases.

5. Compounds according to claim 1, namely compounds of general formula **Id** 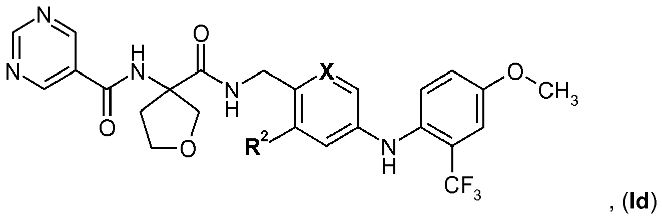 wherein
**R**² denotes H, Cl or F and
**X** denotes CH or N,
the enantiomers, the diastereomers, the mixtures and the salts thereof, particularly the physiologically acceptable salts thereof with organic or inorganic acids or bases.

6. Compounds according to one of the preceding claims, namely 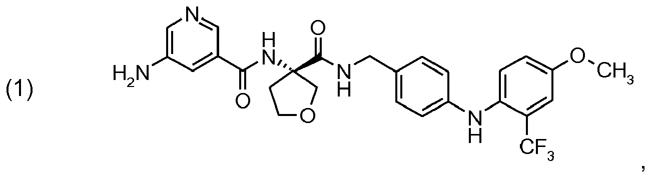 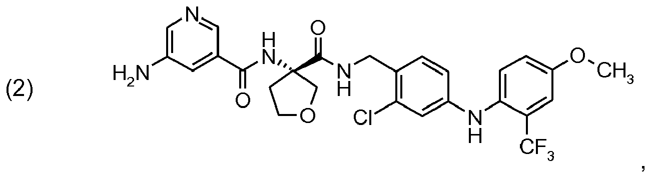 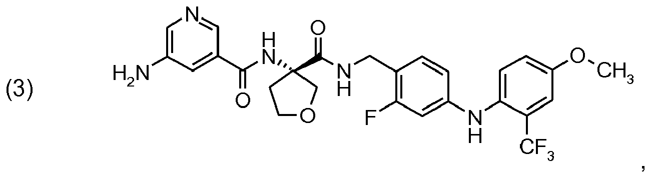 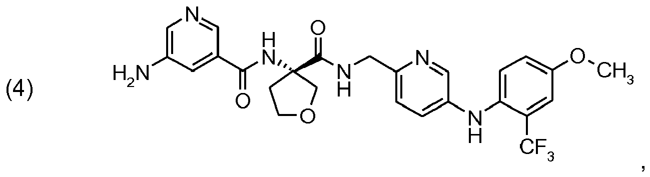 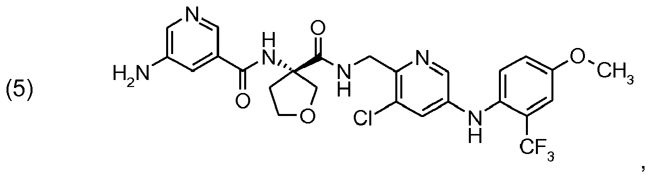 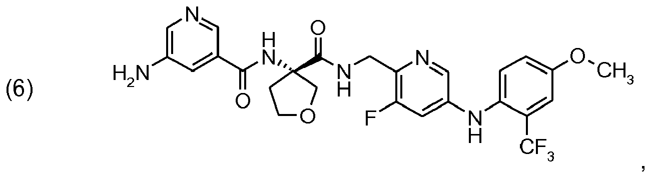 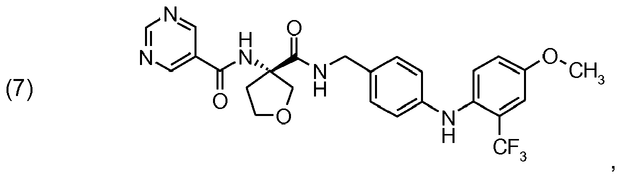 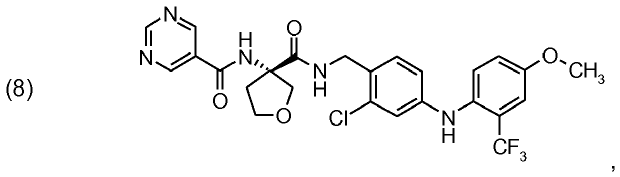 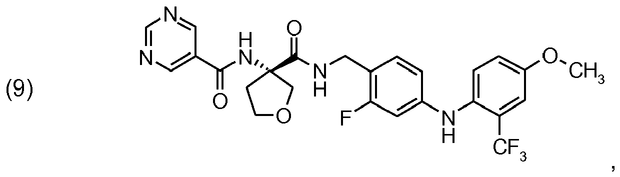 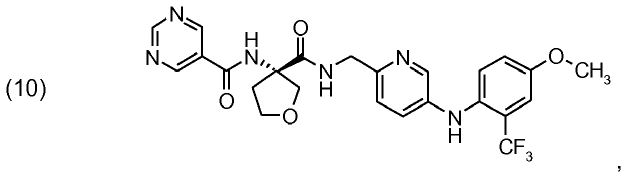 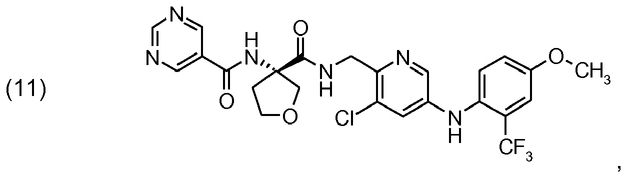 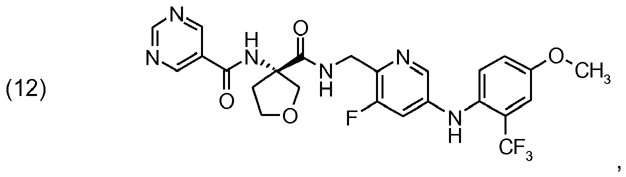 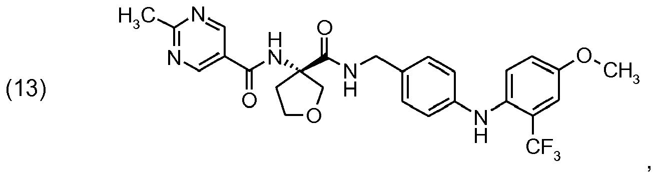 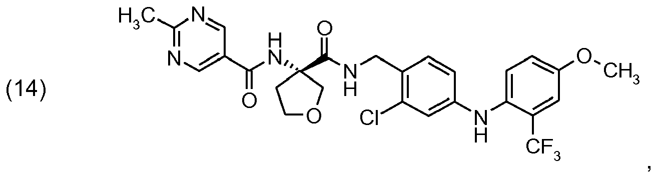 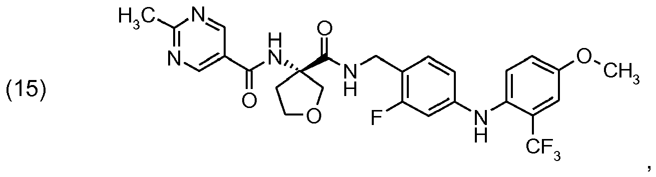 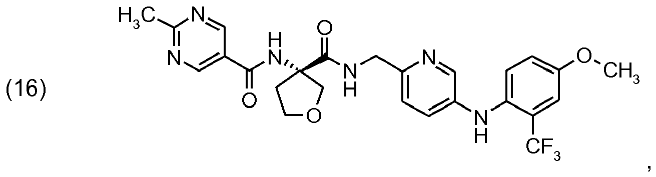 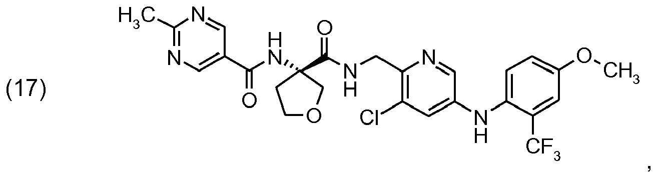 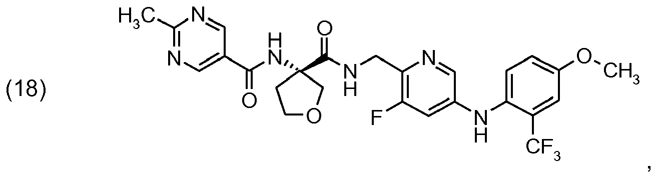 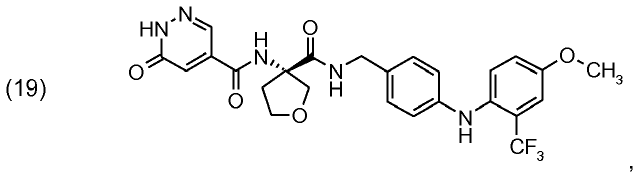 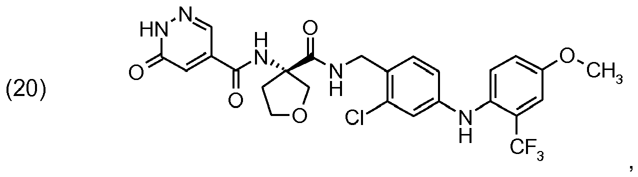 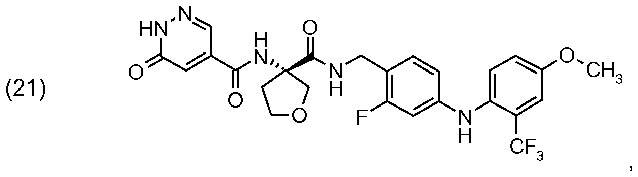 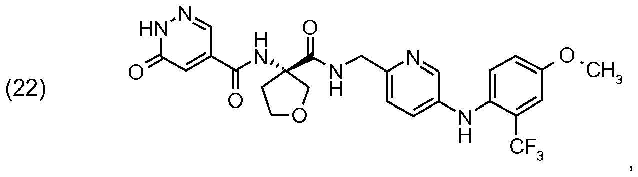 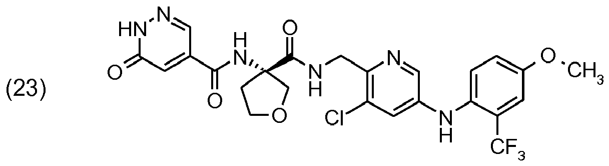 and 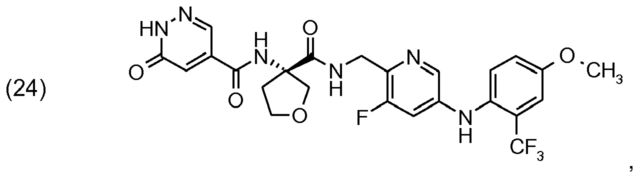 the enantiomers, the diastereomers, the mixtures and the salts thereof, particularly the physiologically acceptable salts thereof with organic or inorganic acids or bases.

7. Physiologically acceptable salts of the compounds according to one of the preceding claims with inorganic or organic acids or bases.

8. Pharmaceutical compositions containing a compound according to one of the preceding claims, optionally together with one or more inert carriers and/or diluents.

9. Medicaments containing a compound according to one of claims 1-7.

10. A compound according to one of claims 1-7 for use in the acute and prophylactic treatment of osteoarthritis, acute pain, visceral pain, neuropathic pain, inflammatory / pain-receptor-mediated pain, tumour pain, headache diseases, diabetic neuropathy, chronic backache, asthma, chronic bronchitis or COPD.

## Revendications

1. Composés de formule générale **I** 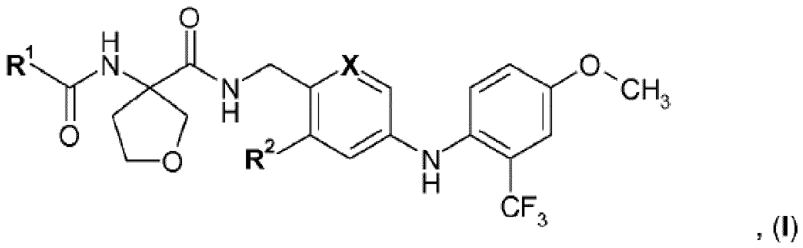 dans laquelle
**R¹** représente un groupe choisi parmi 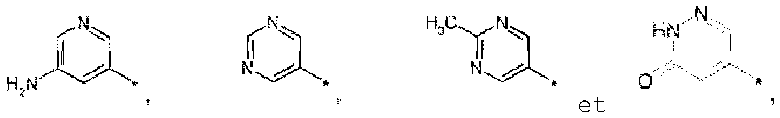
**R²** représente H, Cl ou F et
**X** représente CH ou N,
leurs énantiomères, leurs diastéréomères, leurs mélanges et leurs sels, en particulier leurs sels physiologiquement acceptables avec des acides ou des bases organiques ou inorganiques.

2. Composés selon la revendication 1, à savoir, composés de formule générale **Ia** 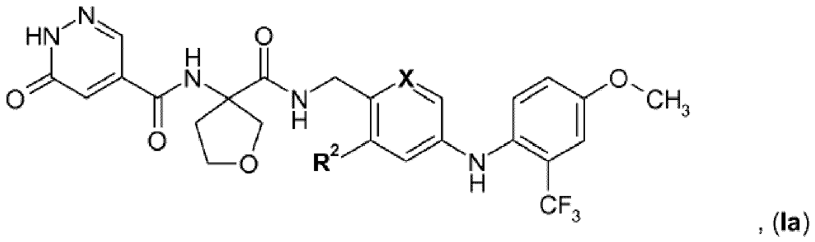 dans laquelle
**R²** représente H, Cl ou F et
**X** représente CH ou N,
leurs énantiomères, leurs diastéréomères, leurs mélanges et leurs sels, en particulier leurs sels physiologiquement acceptables avec des acides ou des bases organiques ou inorganiques.

3. Composés selon la revendication 1, à savoir, composés de formule générale **Ib** 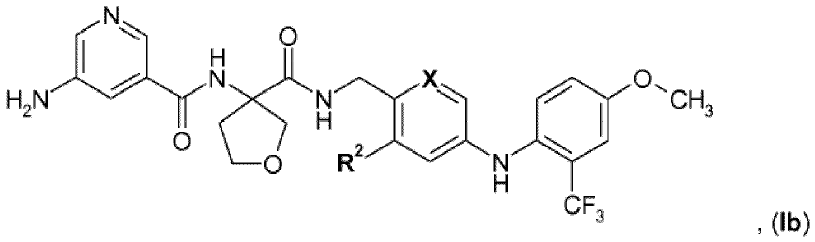 dans laquelle
**R²** représente H, Cl ou F et
**X** représente CH ou N,
leurs énantiomères, leurs diastéréomères, leurs mélanges et leurs sels, en particulier leurs sels physiologiquement acceptables avec des acides ou des bases organiques ou inorganiques.

4. Composés selon la revendication 1, à savoir, composés de formule générale **Ic** 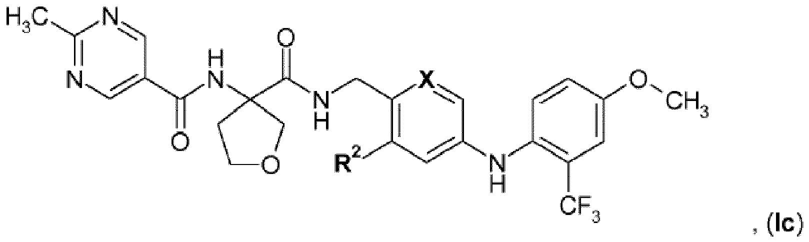 dans laquelle
**R²** représente H, Cl ou F et
**X** représente CH ou N,
leurs énantiomères, leurs diastéréomères, leurs mélanges et leurs sels, en particulier leurs sels physiologiquement acceptables avec des acides ou des bases organiques ou inorganiques.

5. Composés selon la revendication 1, à savoir, composés de formule générale **Id** 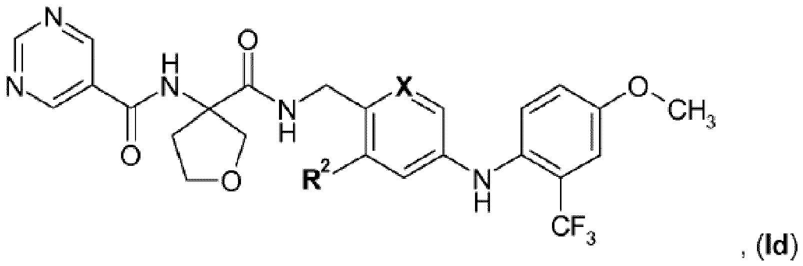 dans laquelle
**R²** représente H, Cl ou F et
**X** représente CH ou N,
leurs énantiomères, leurs diastéréomères, leurs mélanges et leurs sels, en particulier leurs sels physiologiquement acceptables avec des acides ou des bases organiques ou inorganiques.

6. Composés selon l'une des revendications précédentes, à savoir 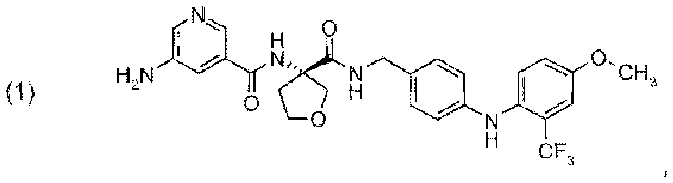 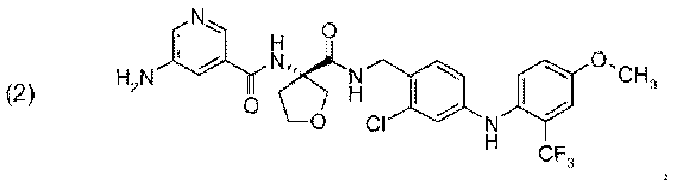 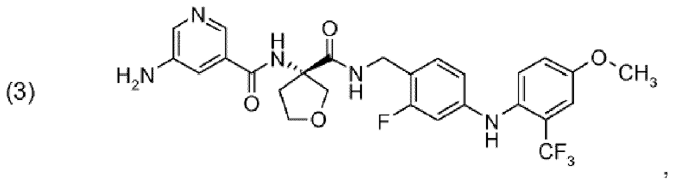 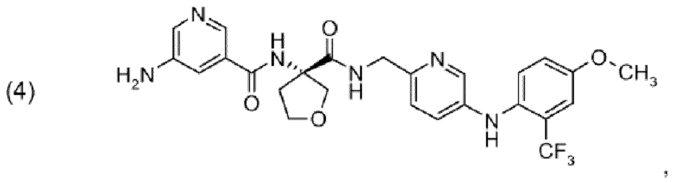 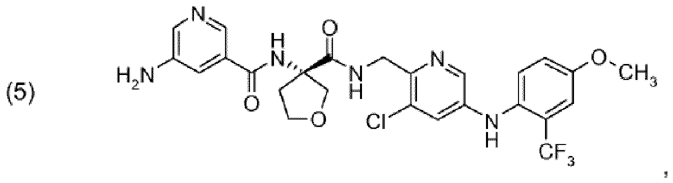 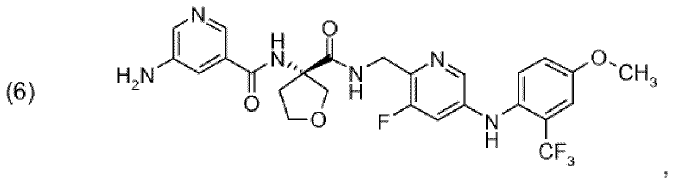 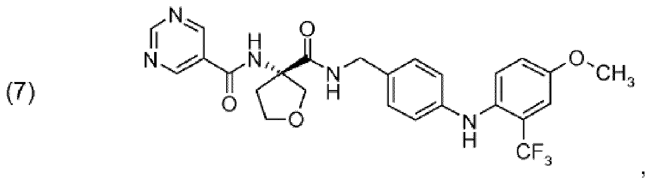 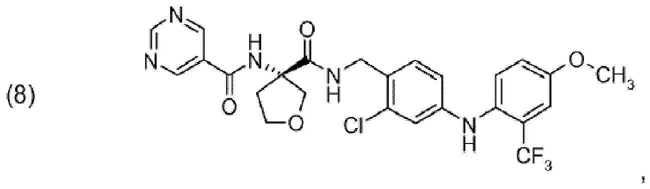 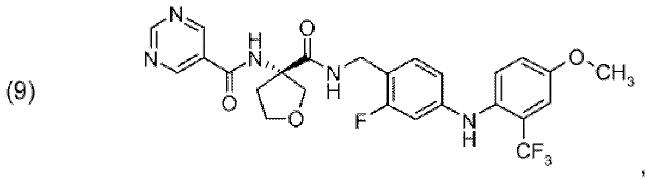 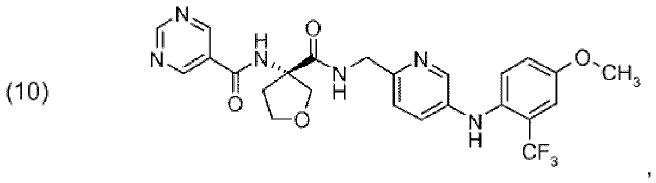 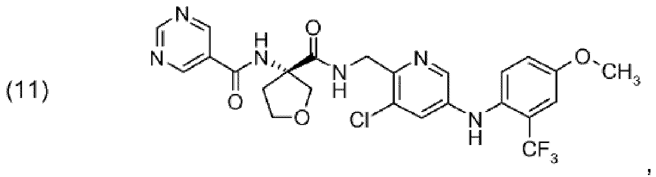 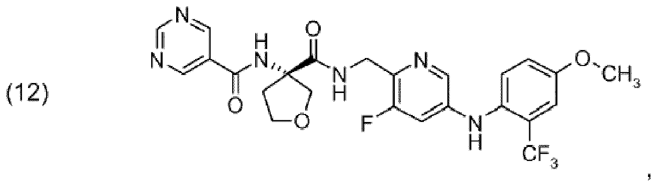 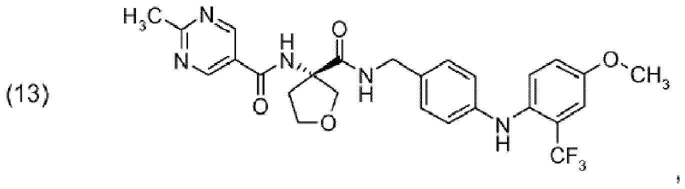 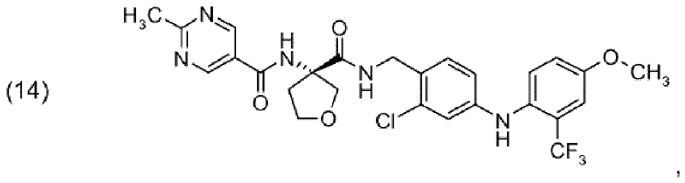 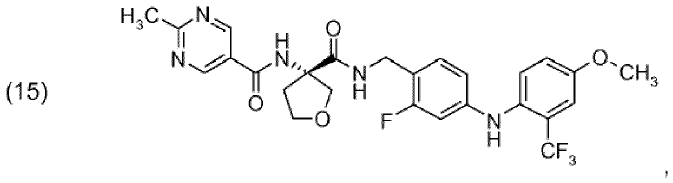 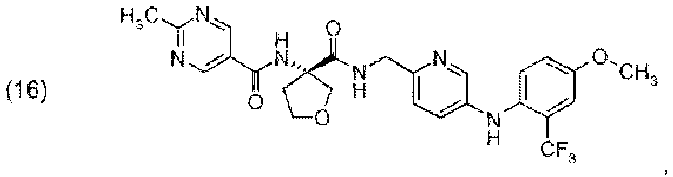 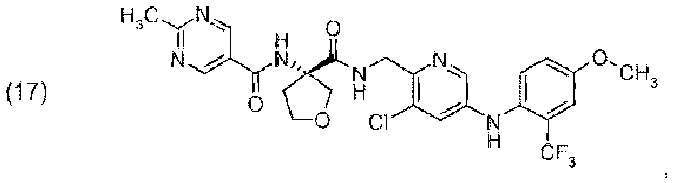 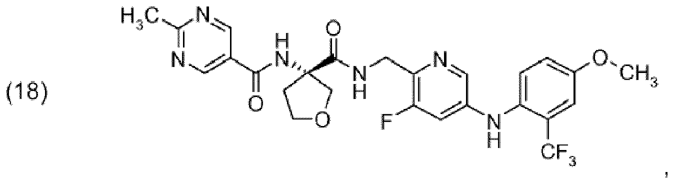 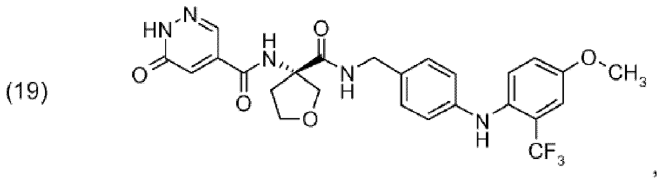 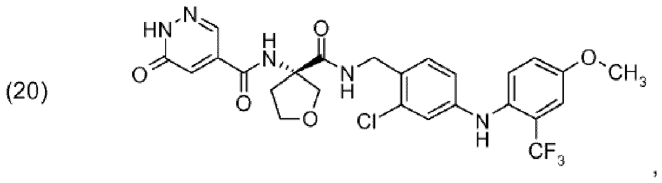 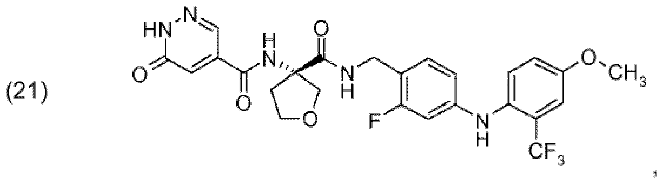 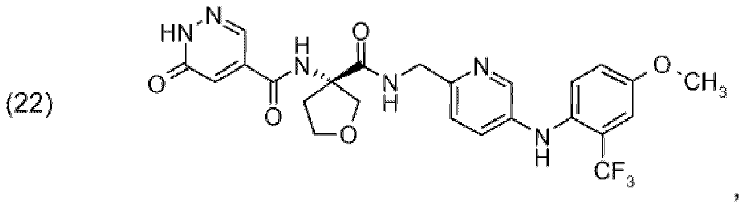 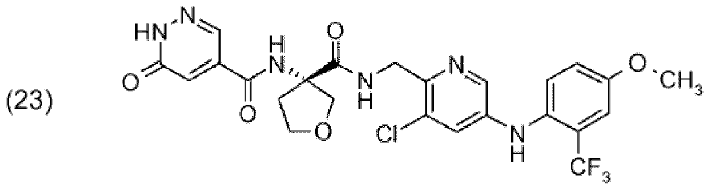 et 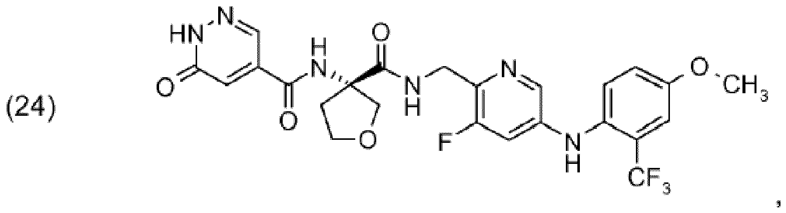 leurs énantiomères, leurs diastéréomères, leurs mélanges et leurs sels, en particulier leurs sels physiologiquement acceptables avec des acides ou des bases organiques ou inorganiques.

7. Sels physiologiquement acceptables des composés selon l'une des revendications précédentes, avec des sels ou des bases inorganiques ou organiques.

8. Compositions pharmaceutiques contenant un composé selon l'une des revendications précédentes, éventuellement, conjointement avec un ou plusieurs véhicules et/ou diluants inertes.

9. Médicament contenant un composé selon l'une des revendications 1 à 7.

10. Composé selon l'une des revendications 1 à 7, pour son utilisation dans le traitement aigu et prophylactique de l'arthrose, de douleurs aiguës, de douleurs viscérales, de douleurs neuropathiques, de douleurs inflammatoires / de douleurs induites par un récepteur nociceptif, de douleurs tumorales, de céphalées, de neuropathie diabétique, de douleurs dorsales chroniques, d'asthme, de bronchite chronique ou de BPCO.
